# EUROPÄISCHE PATENTSCHRIFT

(11) **EP 1 133 500 B1**
(45) Veröffentlichungstag und Bekanntmachung des Hinweises auf die Patenterteilung: **11.09.2002**
(21) Anmeldenummer: 99959292.6
(22) Anmeldetag: 20.11.1999
(51) Int. Cl.: C07F 9/38, A61K 31/66, A01N 57/18, A61P 31/04, A61P 31/12, A61P 33/06, C07F 9/40, C07F 9/58

(54) **PHOSPHORORGANISCHE VERBINDUNGEN UND IHRE VERWENDUNG**
ORGANOPHOSPHOROUS COMPOUNDS AND THE USE THEREOF
COMPOSES PHOSPHORORGANIQUES ET LEUR UTILISATION

(30) Priorität: 25.11.1998 DE 19854403
(43) Veröffentlichungstag der Anmeldung: 19.09.2001
(73) Patentinhaber: Jomaa Pharmaka GmbH, 35392 Giessen (DE)
(72) Erfinder: JOMAA, Hassan Jomaa Pharmaka GmbH, 35392 Giesen (DE)
(74) Vertreter: Panten, Kirsten
(86) Internationale Anmeldenummer: EP9908966
(87) Internationale Veröffentlichungsnummer: WO00031085

(56) Entgegenhaltungen:
- EP-A- 0 009 686
- WO-A-99/52515
- DE-A- 2 733 658
- US-A- 4 693 742
- CHEMICAL ABSTRACTS, vol. 093, no. 19, 10. November 1980 (1980-11-10) Columbus, Ohio, US; abstract no. 186456, KAMIYA T ET AL: "Studies on new phosphonic acid-containing antibiotics: synthesis of FR-31564 and related antibiotics" XP002122512 & CURR. CHEMOTHER. INFECT. DIS., PROC. INT. CONGR. CHEMOTHER., 11TH (43MKAT);1980; VOL.1,; PP.355-8, Fujisawa Pharm. Co., Ltd.;Res. Lab.; Osaka; Japan
- GLABE A R ET AL: "NOVEL FUNCTIONALIZED ACYLPHOPHONATES AS PHOPHONOFORMATE ANALOGS" JOURNAL OF ORGANIC CHEMISTRY,US,AMERICAN CHEMICAL SOCIETY. EASTON, Bd. 61, Nr. 20, Seite 7212-7216 XP000627289 ISSN: 0022-3263
- H C NEU ET AL: "In Vitro and In Vivo Antibacterial Activity of FR-31564, a Phosphonic Acid Antimicrobial Agent" ANTIMICROBIAL AGENTS AND CHEMOTHERAPY,US,AMERICAN SOCIETY FOR MICROBIOLOGY, WASHINGTON, DC, Bd. 19, Nr. 6, Seite 1013-1023-1023 XP002113260 ISSN: 0066-4804
- H C NEU ET AL: "Synergy of Fosmidomycin (FR-31564) and Other Antimicrobial Agents" ANTIMICROBIAL AGENTS AND CHEMOTHERAPY,US,AMERICAN SOCIETY FOR MICROBIOLOGY, WASHINGTON, DC, Bd. 22, Nr. 4, Seite 560-563-563 XP002113261 ISSN: 0066-4804
- D GREENWOOD: "Fosfomycin Trometamol: Activity In Vitro against Urinary Tract Pathogens" INFECTION,DE,MMV MEDIZIN VERLAG, MUENCHEN, Bd. 18, Nr. SUPPL. 02, Seite S60-S64-S64 XP002113262 ISSN: 0300-8126
- CHEMICAL ABSTRACTS, vol. 105, no. 19, 10. November 1986 (1986-11-10) Columbus, Ohio, US; abstract no. 166897, YAMAJI T ET AL: "N-Substituted alkyl amine phosphates as herbicides" XP002122513 & JP 61 106504 A (TEIJIN LTD.;JAPAN)

## Beschreibung

Die Erfindung betrifft phosphororganische Verbindungen sowie ihre Salze, Ester und Amide und ihre Verwendung zur therapeutischen und prophylaktischen Behandlung von Infektionen bei Mensch und Tier, die durch Viren, Bakterien, Pilze und Parasiten hervorgerufen werden, und ihre Verwendung als Fungizid, Bakterizid und Herbizid bei Pflanzen, Erfindungsgemäß umfassen die phosphororganischen Verbindungen Phosphinoylderivate, Phosphinsäurederivate und Phosphonsäurederivate.

Es besteht ein starker Bedarf, für die Bereicherung der Behandlung von Mensch und Tier sowie den Schutz von Pflanzen Mittel bereitzustellen, die nicht nur eine starke Wirksamkeit besitzen, sondern auch im Gegensatz zu anderen Arzneimitteln bzw. Pflanzenschutzmitteln verringerte Nebenwirkungen zeigen und damit eine geringere Gesundheitsgefahr für den Menschen bedeuten.

Aus der DE 27 33 658 A sind bereits Phosphonsäurederivate der Formel R₁-N(OR₂)-A-P(O)(OH)₂ bekannt, worin R₁ für Wasserstoff oder Acyl steht, R₂ für Wasserstoff, Ar-Niederalkyl oder Acyl steht und A eine niedere Alkylen-, niedere Alkenylen oder niedere Hydroxyalkylengruppe, vorzugsweise Formyl, Acety oder Trimethylen ist, sowie deren Ester und pharmazeutischen Salze. Diese Verbindungen werden für die therapeutische Behandlung von Infektionen eingesetzt, die durch Bakterien hervorgerufen werden.

Aufgabe der vorliegenden Erfindung ist es daher, alternative Substanzen bereitzustellen, die bei Infektion durch Bakterien, aber auch bei Infektionen durch Viren, Pilze und Parasiten bei Menschen und Tieren und als Fungizid, Bakterizid und Herbizid bei Pflanzen einsetzbar sind und die oben angegebenen Bedingungen erfüllen.

Diese Aufgabe wird in völlig überraschender Weise durch die in Anspruch 1 definierte Stoffgruppe gelöst. Diese Stoffgruppe zeigt eine antiinfektiöse Wirkung gegen Viren, Bakterien, Pilze, ein- und mehrzellige Parasiten.

Die erfindungsgemäßen phosphororganischen Verbindungen entsprechen der allgemeinen Formel (I): 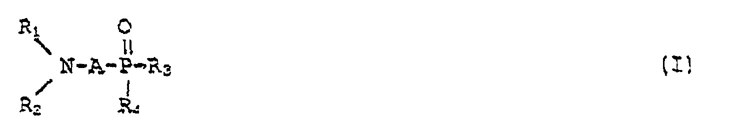 in der R₁ und R₂ gleich oder verschieden sind und aus der Gruppe ausgewählt sind, die aus Wasserstoff, substituiertem und unsubstituiertem C₁₋₉-Alkyl, substituiertem und unsubstituiertem Hydroxy-C₁₋₉-alkyl, substituiertem und unsubstituiertem C₁₋₉-Alkenyl, substituiertem und unsubstituiertem C₁₋₉-Alkinyl, substituiertem und unsubstituiertem Aryl, substituiertem und unsubstituiertem Acyl, substituiertem und unsubstituiertem Cycloalkyl, substituiertem und unsubstituiertem Aralkyl, substituiertem und unsubstituiertem heterocyclischen Rest, Halogen, OX₁ und OX₂ besteht,
wobei X₁ und X₂ gleich oder verschieden sein können und aus der Gruppe ausgewählt sind, die aus Wasserstoff, substituiertem und unsubstituiertem C₁₋₉-Alkyl, substituiertem und unsubstituiertem Hydroxy-C₁₋₉-alkyl, substituiertem und unsubstituiertem C₁₋₉-Alkenyl, substituiertem und unsubstituiertem C₁₋₉-Alkinyl, substituiertem und unsubstituiertem Aryl, substituiertem und unsubstituiertem Acyl, substituiertem und unsubstituiertem Cycloalkyl, substituiertem und unsubstituiertem Aralkyl, substituiertem und unsubstituiertem heterocyclischen Rest besteht,
in der A der folgenden Formel (II) entspricht: 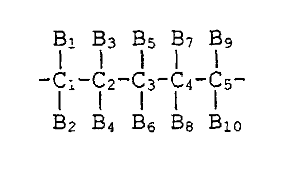 wobei ein oder mehrere der Kohlenstoffatome, ausgewählt aus der Gruppe C₃, C₄, C₅, mitsamt ihren Substituenten auch wegfallen können, und mindestens ein vorliegender Substituent von B₁ bis B₁₀ eine C₃₋₈-Cycloalkyl-(C₀₋₉)-alkylgruppe ist, wobei sowohl die C₃₋₈-Cycloalkylgruppe als auch die C₀₋₉-Alkylgruppe ein oder mehrere Doppelbindungen aufweisen können und ein oder zwei Kohlenstoffatome der Cycloalkylgruppe durch Stickstoff-, Sauerstoff- oder Schwefelatome ersetzt sein können, und wobei sowohl die Cycloalkylgruppe als auch die Alkylgruppe mit Hydroxy-, Halogen-, Amino-, Oxogruppen mit verzweigten oder unverzweigten C₁₋₉-Alkylgruppen und C₂₋₉-Alkenylgruppen substituiert sein können, wobei die C₁₋₉-Alkylgruppen und C₂₋₉-Alkenylgruppen mit Wasserstoff-, Hydroxy-, Amino-, Halogen- und Oxogruppen substituiert sein können, und die restlichen vorliegenden Substituenten B₁ bis B₁₀ aus der Gruppe ausgewählt sind, die aus Wasserstoff, Hydroxy-, Halogen-, Aminogruppen, C₁₋₂₆-Alkylresten, C₁₋₂₆-Alkoxyresten, C₁₋₂₆-Alkoxy-C₁₋₂₆-Alkylresten besteht oder beide Substituenten eines C-Atoms zusammen eine Oxogruppe bilden, wobei jeder C₁₋₂₆-Alkylrest und jeder C₁₋₂₆-Alkoxyrest verzweigt oder unverzweigt und gesättigt oder mit ein oder mehreren Doppelbindungen ungesättigt sein kann und mit Hydroxy-, Amino-, Halogenund Oxogruppen substituiert sein kann,
in der R₃ und R₄ gleich oder verschieden sind und aus der Gruppe ausgewählt ist, die aus substituiertem und unsubstituiertem C₁₋₂₆-Alkyl, Hydroxy-C₁₋₂₆-alkyl, substituiertem und unsubstituiertem Aryl, substituiertem und unsubstituiertem Acyl, substituiertem und unsubstituiertem Aralkyl, substituiertem und unsubstituiertem C₁₋₂₆-Alkenyl, substituiertem und unsubstituiertem C₁₋₂₆-Alkinyl, substituiertem und unsubstituiertem Cycloalkyl, substituiertem und unsubstituiertem heterocyclischem Rest, Halogen, OX₃ und OX₄ besteht,
wobei X₃ und X₄ gleich oder verschieden sind und aus der Gruppe ausgewählt sind, die aus Wasserstoff, substituiertem und unsubstituiertem C₁₋₂₆-Alkyl, substituiertem und unsubstituiertem Hydroxy-C₁₋₂₆-alkyl, substituiertem und unsubstituiertem Aryl, substituiertem und unsubstituiertem Aralkyl, substituiertem und unsubstituiertem C₁₋₂₆-Alkanyl, substituiertem und unsubstituiertem C₁₋₂₆-Alkinyl, substituiertem und unsubstituiertem Cycloalkyl, substituiertem und unsubstituiertem heterocyclischem Rest, einem Silyl, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, besteht, und deren pharmazeutisch akzeptablen Salze, Ester und Amide und Salze der Ester.

Insbesondere eignen sich die Verbindungen, die die folgende Formel (III) haben: 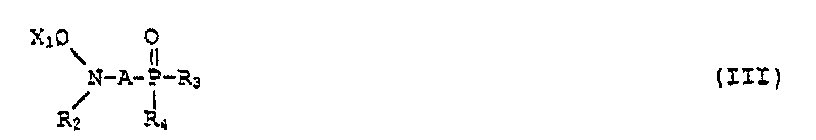 wobei X₁ bevorzugt ein Wasserstoff ist und R₂ ein Acylrest ist, besonders bevorzugt ein Formylrest oder Acetylrest ist.

Bevorzugt sind X₃ und X₄ OX₃ und OX₄, und X₃ und X₄ Wasserstoff, ein Metall der ersten, zweiten oder dritten Hauptgruppe des Periodensystems, Ammonium, substituiertes Ammonium, oder Ammoniumverbindungen, die sich von Ethylendiamin oder Aminosäuren ableiten. D.h es werden die Salzverbindungen der phosphororganischen Verbindungen mit organischen oder anorganischen Basen (z.B. Natriumsalz, Kaliumsalz, Calciumsalz, Aluminiumsalz, Ammoniumsalz, Magnesiumsalz, Triethylaminsalz, Ethanolaminsalz, Dicyclohexylaminsalz, Ethylendiaminsalz, N,N'-Dibenzylethylen-diaminsalz etc.) sowie Salze mit Aminosäuren (z.B. Arginin-salz, Asparaginsäuresalz, Glutaminsäuresalz etc.) und dergleichen gebildet.

Bevorzugt besteht die Kohlenstoffkette von A mit der Formel (II) aus drei Kohlenstoffatomen C₁, C₂, C₃.

Ebenfalls sind Verbindungen bevorzugt, in denen die Kohlenstoffkette von A mit der Formel (II) aus vier Kohlenstoffatomen C₁, C₂, C₃, C₄ besteht und B₇ oder B₈ oder beide eine Hydroxygruppe sind. In diesem Fall sind für R₃ und R₄ auch Methylengruppen bevorzugt.

Bevorzugt ist ferner, daß B₁ und B₂ zusammen eine Oxogruppe bilden. In diesem Fall besteht die Kohlenstoffkette in A aus den vier Kohlenstoffatomen C₁, C₂, C₃, C₄.

Bevorzugt ist ferner, daß B₇ und B₈ zusammen eine Oxogruppe bilden. In diesem Fall besteht die Kohlenstoffkette in A ebenfalls aus den vier Kohlenstoffatomen C₁, C₂, C₃, C₄.

Die Kohlenstoffkette besteht bevorzugt aus den 5 Kohlenstoffatomen C₁, C₂, C₃, C₄, C₅, wobei B₁ und B₂ zusammen eine Oxogruppe bilden und mindestens ein Substituent von B₉ oder B₁₀ eine Hydroxylgruppe ist oder B₉ und B₁₀ zusammen ebenfalls eine Oxogruppe bilden.

Besonderheiten der obigen Definitionen und geeignete Beispiele dafür werden nachfolgend angegeben:
"Acyl" ist ein Substituent, der von einer Säure stammt, wie von einer organischen Carbonsäure, Kohlensäure, Carbaminsäure oder der den einzelnen vorstehenden Säuren entsprechenden Thiosäure oder Imidsäure, oder von einer organischen Sulfonsäure, wobei diese Säuren jeweils aliphatische, aromatische und/oder heterocyclische Gruppen im Molekül umfassen sowie Carbamoyl oder Carbamimidoyl.

Geeignete Beispiele für diese Acylgruppen werden nachfolgend angegeben.

Als aliphatische Acylgruppen werden von einer aliphatischen Säure stammende Acylreste bezeichnet, zu denen die folgenden gehören:
Alkanoyl (z.B. Formyl, Acetyl, Propionyl, Butyryl, Isobutyryl, Valeryl, Isovaleryl, Pivaloyl etc.); Alkenoyl (z. B. Acryloyl, Methacryloyl, Crotonoyl etc.); Alkylthioalkanoyl (z.B. Methylthioacetyl, Ethylthioacetyl etc.) Alkansulfonyl (z.B. Mesyl, Ethansulfonyl, Propansulfonyl etc.); Alkoxycarbonyl (z.B. Methoxycarbonyl, Ethoxycarbonyl, Propoxycarbonyl, Isopropoxycarbonyl, Butoxycarbonyl, Isobutoxycarbonyl etc.); Alkylcarbamoyl (z.B. Methylcarbamoyl etc.); (N-Alkyl)-thiocarbamoyl (z.B. (N-Methyl)-thiocarbamoyl etc.); Alkylcarbamimidoyl (z.B. Methylcarbamimidoyl etc.); Oxalo; Alkoxalyl (z.B. Methoxalyl, Ethoxalyl, Propoxalyl etc.).

Bei den obigen Beispielen fiir aliphatische Acylgruppen kann der aliphatische Kohlenwasserstoffteil, insbesondere die Alkylgruppe bzw. der Alkanrest, ggf. einen oder mehrere geeignete Substituenten aufweisen, wie Amino, Halogen (z.B. Fluor, Chlor, Brom etc.), Hydroxy, Hydroxyimino, Carboxy, Alkoxy (z.B. Methoxy, Ethoxy, Propoxy etc.), Alkoxycarbonyl, Acylamino (z.B. Benzyloxycarbonylamino etc.), Acyloxy (z.B. Acetoxy, Benzoyloxy etc.) und dergleichen; als bevorzugte aliphatische Acylreste mit solchen Substituenten sind z.B. mit Amino, Carboxy, Amino und Carboxy, Halogen, Acylamino oder dergleichen substituierte Alkanoyle zu nennen.

Als aromatische Acylreste werden solche Acylreste bezeichnet, die von einer Säure mit substituierter oder nicht substituierter Arylgruppe stammen, wobei die Arylgruppe Phenyl, Toluyl, Xylyl, Naphthyl und dergleichen umfassen kann; geeignete Beispiele werden nachfolgend angegeben:
Aroyl (z.B. Benzoyl, Toluoyl, Xyloyl, Naphthoyl, Phthaloyl etc.); Aralkanoyl (z.B. Phenylacetyl etc.); Aralkenoyl (z.B. Cinnamoyl etc.); Aryloxyalkanoyl (z.B. Phenoxyacetyl etc.); Arylthioalkanoyl (z.B. Phenylthioacetyl etc.); Arylaminoalkanoyl (z.B. N-Phenylglycyl, etc.); Arensulfonyl (z.B.Benzolsulfonyl, Tosyl bzw. Toluolsulfonyl, Naphthalinsulfonyl etc.); Aryloxycarbonyl (z.B. Phenoxycarbonyl, Naphthyl-oxycarbonyl etc.); Aralkoxycarbonyl (z.B. Benzyloxycarbonyl etc.); Arylcarbamoyl (z.B. Phenylcarbamoyl, Naphthylcarbamoyl etc.); Arylglyoxyloyl (z.B. Phenylglyoxyloyl etc.).

Bei den vorstehenden Beispielen für aromatische Acylreste kann der aromatische Kohlenwasserstoffteil (insbesondere der Arylrest) und/oder der aliphatische Kohlenwasserstoffteil (insbesondere der Alkanrest) ggf ein oder mehrere geeignete Substituenten aufweisen, wie solche, die als geeignete Substituenten für die Alkylgruppe bzw. den Alkanrest bereits angegeben wurden. Insbesondere und als Beispiel für bevorzugte aromatische Acylreste mit besonderen Substituenten werden mit Halogen und Hydroxy oder mit Halogen und Acyloxy substituiertes Aroyl und mit Hydroxy, Hydroxyimino, Dihalogenalkanoyloxyimino substituiertes Aralkanoyl angegeben sowie
Arylthiocarbamoyl (z.B. Phenylthiocarbamoyl etc.);
Arylcarbamimidoyl (z.B. Phenylcarbamimidoyl etc.).

Als heterocyclischer Acylrest wird ein Acylrest verstanden, der von einer Säure mit heterocyclischer Gruppe stammt; dazu gehören:
Heterocyclisches Carbonyl, bei dem der heterocyclische Rest ein aromatischer oder aliphatischer 5-bis 6-gliedriger Heterocyclus mit zumindest einem Heteroatom aus der Gruppe Stickstoff, Sauerstoff und Schwefel ist (z.B. Thiophenyl, Furoyl, Pyrrolcarbonyl, Nicotinoyl etc.);
Heterocyclus-Alkanoyl, bei dem der heterocyclische Rest 5- bis 6-gliedrig ist und zumindest ein Heteroatom aus der Gruppe Stickstoff, Sauerstoff und Schwefel aufweist (z.B. Thiophenyl-acetyl, Furylacetyl, Imidazolylpropionyl, Tetrazolylacetyl, 2-(2-Amino-4-thiazolyl)-2-methoxyiminoacetyl etc.) und dergleichen.

Bei den obigen Beispielen für heterocyclische Acylreste kann der Heterocyclus und/oder der aliphatische Kohlenwasserstoffteil ggf. einen oder mehrere geeignete Substituenten aufweisen, wie die gleichen, die als geeignet für Alkyl- und Alkangruppen angegeben wurden.

"Alkyl" ist ein gerad- oder verzweigtkettiger Alkylrest, wie Methyl, Ethyl, Propyl, Isopropyl, Butyl, Isobutyl, tert.-Butyl, Pentyl, Hexyl und dergleichen.

"Hydroxylalkyl" ist ein gerad- oder verzweigtkettiger Alkylrest, der mindestens eine Hydroxylgruppe aufweist, bevorzugt ein oder zwei Hydroxylgruppen.

Zu "Alkenyl" gehören gerad- oder verzweigtkettige Alkenylgruppen, wie z.B. Vinyl, Propenyl (z.B. 1-Propenyl, 2-Propenyl), 1-Methylpropenyl, 2-Methylpropenyl, Butenyl, 2-Ethylpropenyl, Pentenyl, Hexenyl.

Zu "Alkinyl" gehören gerad- oder verzweigtkettige Alkinylgruppen.

Cycloalkyl steht vorzugsweise für ein ggfs. substituiertes C₃₋₈-Cycloalkyl; als mögliche Substituenten sind u.a. Alkyl, Alkenyl, Alkinyl, Alkoxy (z.B. Methoxy, Ethoxy etc.), Halogen (z.B. Fluor, Chlor, Brom etc.), Nitro und dergleichen geeignet.

Aryl ist ein aromatischer Kohlenwasserstoffrest, wie Phenyl Naphthyl usw., der ggf. einen oder mehrere geeignete Substituenten aufweisen kann, wie Alkyl, Alkenyl, Alkinyl, Alkoxy (z.B. Methoxy, Ethoxy etc.), Halogen (z.B. Fluor, Chlor, Brom etc.), Nitro und dergleichen.

Zu "Aralkyl" gehören Mono-, Di-, Triphenylalkyle wie Benzyl, Phenethyl, Benzhydryl, Trityl und dergleichen, wobei der aromatische Teil ggf. ein oder mehrere geeignete Substituenten aufweisen kann wie Alkoxy (z.B. Methoxy, Ethoxy etc.), Halogen (z.B. Fluor, Chlor, Brom etc.), Nitro und dergleichen.

Vorzugsweise können die Reste X₃ und X₄ so gewählt werden, daß Ester an der Phosphinogruppe oder Phosphonogruppe gebildet werden. Zu geeigneten Beispielen für solche Ester gemäß der Formeln (I) und (III) zählen geeignete Mono- und Diester, und zu bevorzugten Beispielen fiir solche Ester gehören Alkylester (z.B. Hexadecanylester, Octadecanylester etc.);
Aralkylester (Benzylester, Phenethylester, Benzhydrylester, Tritylester etc.);
Arylester (z.B. Phenylester, Tolylester, Naphthylester etc.); Aroylalkylester (z.B. Phenacylester etc.); und Silylester (z.B. von Trialkylhalogensilyl, Dialkyldihalogensilyl, Alkyltrihalogensilyl, Dialkylarylhalogensilyl, Trialkoxyhalogensilyl, Dialkylaralkylhalogensilyl, Dialkoxydihalogensilyl, Trialkoxyhalogensilyl etc.) und dergleichen.

Beim obigen Ester kann der Alkan- und/oder Arenteil wahlweise zumindest einen geeigneten Substituenten aufweisen wie Halogen, Alkoxy, Hydroxy, Nitro oder dergleichen.

Die erfindungsgemäßen Verbindungen gemäß der Formeln (I) und (III) können in ihrer protonierten Form als Ammoniumsalz organischer oder anorganischer Säuren, wie Salzsäure, Bromwasserstoffsäure, Schwefelsäure, Salpetersäure, Methansulfonsäure, p-Toluolsulfonsäure, Essigsäure, Milchsäure, Maleinsäure, Fumarsäure, Oxalsäure, Weinsäure, Benzoesäure, etc. vorliegen.

Die erfindungsgemäßen Verbindungen der Formeln (I) und (III) lassen beispielsweise für Doppelbindungen enthaltende oder chirale Gruppen R₁, R₂, R₃, R₄, X₁, X₂, X₄ oder A das Auftreten räumlicher Isomerer zu. Die erfindungsgemäße Verwendung der Verbindungen umfaßt alle räumlichen Isomere sowohl als Reinstoffe als auch in Form ihrer Mischungen,

Die phosphororganischen Verbindungen sind insbesondere für die therapeutische und prophylaktischen Behandlung von Infektionen bei Mensch und Tier geeignet, die durch Viren, Bakterien, ein- und mehrzellige Parasiten und Pilze hervorgerufen werden.

Die Verbindungen sind gegen einzellige Parasiten (Protozoen) wirksam, insbesondere gegen Erreger der Malaria und der Schlafkrankheit sowie der Chagas-Krankheit, der Toxoplasmose, der Amöbenruhr, der Leishmaniosen, der Trichomoniasis, der Pneumozystose, der Balantidiose, der Kryptosporidiose, der Sarkozystose, der Akanthamöbose, der Naeglerose, der Kokzidiose, der Giardiose und der Lambliose.

Sie sind daher insbesondere als Malariaprophylaxe und als Prophylaxe der Schlafkrankheit sowie der Chagas-Krankheit, der Toxoplasmose, der Amöbenruhr, der Leishmaniosen, der Trichomoniasis, der Pneumozystose, der Balantidiose, der Kryptosporidiose, der Sarkozystose, der Akanthamöbose, der Naeglerose, der Kokzidiose, der Giardiose und der Lambliose geeignet.

Die erfindungsgemäßen Wirkstoffe sind insbesondere gegen die folgenden Bakterien einsetzbar:
Bakterien der Familie Propionibacteriaceae, insbesondere der Gattung Propionibacterium, insbesondere die Art Propionibacterium acnes, Bakterien der Familie Actinomycetaceae, insbesondere der Gattung Actinomyces, Bakterien der Gattung Corynebacterium, insbesondere die Arten Corynebacterium diphteriae und Corynebacterium pseudotuberculosis, Bakterien der Familie Mycobacteriaceae, der Gattung Mycobacterium, insbesondere die Arten Mycobacterium leprae, Mycobacterium tuberculosis, Mycobacterium bovis und Mycobacterium avium, Bakterien der Familie Chlamydiaceae, insbesondere die Spezies Chlamydia trachomatis und Chlamydia psittaci, Bakterien der Gattung Listeria, insbesondere die Art Listeria monocytogenes, Bakterien der Art Erysipelthrix rhusiopathiae, Bakterien der Gattung Clostridium, Bakterien der Gattung Yersinia, der Spezies Yersinia pestis, Yersinia pseudotuberculosis, Yersinia emerocolitica und Yersinia ruckeri, Bakterien der Familie Mycoplasmataceae, der Gattungen Mycoplasma und Ureaplasma, insbesondere die Art Mycoplasma pneumoniae, Bakterien der Gattung Brucella, Bakterien der Gattung Bordetella, Bakterien der Familie Neisseriaceae, insbesondere der Gattungen Neisseria und Moraxella, insbesondere die Arten Neisseria meningitides, Neisseria gonorrhoeae und Moraxella bovis, Bakterien der Familie Vibrionaceae, insbesondere der Gattungen Vibrio, Aeromonas, Plesiomonas und Photobacterium, insbesondere die Arten Vibrio cholerae, Vibrio anguillarum und Aeromonas salmonicidas, Bakterien der Gattung Campylobacter, insbesondere die Arten Campylobacter jejuni, Campylobacter coli und Campylobacter fetus, Bakterien der Gattung Helicobacter, insbesondere die Art Helicobacter pylori, Bakterien der Familien Spirochaetaceae und der Leptospiraceae, insbesondere der Gattungen Treponema, Borrelia und Leptospira, insbesondere Borrelia burgdorferi, Bakterien der Gattung Actinobacillus, Bakterien der Familie Legionellaceae, der Gattung Legionella, Bakterien der Familie Rickettsiaceae und Familie Bartonellaceae, Bakterien der Gattungen Nocardia und Rhodococcus,Bakterien der Gattung Dermatophilus, Bakterien der Familie Pseudomonadaceae, insbesondere der Gattungen Pseudomonas und Xahthomonas, Bakterien der Familie Enterobacteriaceae, insbesondere der Gattungen Escherichia, Klebsiella, Proteus, Providencia, Salmonella, Serratia und Shigella, Bakterien der Familie Pasteurellaceae, insbesondere der Gattung Haemophilus, Bakterien der Familie Micrococcaceae, insbesondere der Gattungen Micrococcus und Staphylococcus, Bakterien der Familie Streptococcaceae, insbesondere der Gattungen Streptococcus und Enterococcus und Bakterien der Familie Bacillaceae, insbesondere der Gattungen Bacillus und Clostridium.

Damit eignen sich phosphororganischen Verbindungen und ihre Derivate zur Behandlung der Diphterie, der Acne vulgaris, der Listeriosen, des Rotlaufs bei Tieren, der Gasbrand beim Mensch und beim Tier, Pararauschbrand bei Mensch und Tier, Tuberkulose bei Mensch und Tier, Lepra, und weitere Mykobacteriosen bei Mensch und Tier, der Paratuberkulose der Tiere, Pest, mesenterialen Lymphadenitis und Pseudotuberkulose bei Mensch und Tier, Cholera, Legionärskrankheit, Borreliose bei Mensch und Tier, Leptospirosen bei Mensch und Tier, Syphilis, Campylobacter-Enteritiden bei Mensch und Tier, Moraxella-Keratokonjunc-tivitis und Serositis der Tiere, Brucellosen der Tiere und des Menschen, Milzbrand bei Mensch und Tier, Aktinomykose bei Mensch und Tier, Streptotrichosen, Psittakose/Ornithose bei Tieren, Q-Fieber, Ehrlichiose.

Weiter ist der Einsatz nützlich bei der Helicobacter-Eradikationstherapie bei Ulcera des Magendarmtraktes.

Es können auch Kombinationen mit einem weiteren Antibiotikum zur Behandlung der obengenannten Erkrankungen eingesetzt werden. Für Kombinationspräparate mit anderen Antiinfektiva eignen sich insbesondere Isoniazid, Rifampicin, Ethambutol, Pyrazinamid, Streptomycin, Protionamid und Dapson zur Behandlung der Tuberkulose.

Die erfindungsgemäßen Wirkstoffe sind ferner insbesondere bei Infektionen mit folgenden Viren einsetzbar:
Parvoviridae: Parvoviren, Dependoviren, Densoviren, Adenoviridae: Adenoviren, Mastadenoviren, Aviadenoviren, Papovaviridae: Papovaviren, insbesondere Papillomaviren (sogenannte Warzenviren), Polyomaviren, insbesondere JC-Virus, BK-Virus, und Miopapovaviren, Herpesviridae: Alle Herpesviren, insbesondere Herpes-Simplex-Viren, der Varizellen/Zoster-Viren, menschlicher Zytomegalievirus, Epstein-Barr-Viren, alle humanen Herpesviren, humanes Herpesvirus 6, Humanes Herpesvirus 7, humanes Herpesvirus 8, Poxviridae:Pockenviren, Orthopox-, Parapox-, Molluscum-Contagiosum-Virus, Aviviren, Capriviren, Leporipoxviren, alle primär hepatotropen Viren, Hepatitisviren: Hepatitis-A-Viren, Hepatitis-B-Viren, Hepatitis-C-Viren, Hepatitis-D-Viren, Hepatitis-E-Viren, Hepatitis-F-Viren, Hepatits-G-Viren, Hepadnaviren: sämtliche Hepatitisviren, Hepatitis-B-Virus, Hepatitis-D-Viren, Picornaviridae: Picornaviren, alle Enteroviren, alle Polioviren, alle Coxsackieviren, alle Echoviren, alle Rhinoviren, Hepatitis-A-Virus, Aphthoviren, Calciviridae: Hepatitis-E-Viren, Reoviridae: Reoviren, Orbiviren, Rotaviren, Togaviridae: Togaviren, Alphaviren, Rubiviren, Pestiviren, Rubellavirus, Flaviviridae: Flaviviren, FSME-Virus, Hepatitis-C-Virus, Orthomyxoviridae: Alle Influenzaviren, Paramyxoviridae: Paramyxoviren, Morbillivirus, Pneumovirus, Masernvirus, Mumpsvirus, Rhabdoviridae: Rhabdoviren, Rabiesvirus, Lyssavirus, viskuläres Stomatitisvirus, Coronaviridae: Coronaviren, Bunyaviridae: Bunyaviren, Nairovirus, Phlebovirus, Uukuvirus, Hantavirus, Hantaanvirus, Arenaviridae: Arenaviren, lymphozytäres Choriomeningitis-Virus, Retroviridae: Retroviren, alle HTL-Viren, humanes T-cell Leukämievirus, Oncornaviren, Spumaviren, Lentiviren, alle HI-Viren, Filoviridae: Marburg- und Ebolavirus, Slow-virus-Infektionen, Prionen, Onkoviren und Leukämieviren.

Die erfindungsgemäßen phosphororganischen Verbindungen sind somit zur Bekämpfung folgender viraler Infekte geeignet:
Eradikation von Papillomaviren zur Vorbeugung von Tumoren, insbesondere von Tumoren der Geschlechtsorgane verursacht durch Papillomaviren beim Menschen, Eradikation von JC-Viren und BK-Viren, Eradikation von Herpesviren, Eradikation humaner Herpesviren 8 zur Behandlung der Kaposi-Sarkoma, Eradikation von Zytomegalie-Viren vor Transplantationen, Eradikation von Eppstein-Barr-Viren vor Transplantation und zur Vorbeugung von Eppstein-Barr-Viren-assozierten Tumoren, Eradikation von Hepatitisviren zur Behandlung von chronischen Leber-Erkrankungen und zur Vorbeugung von Lebertumoren und Leberzirrhosen, Eradikation von Coxsackieviren bei Kardiomyopathien, Eradikation von Coxsackieviren bei Diabetes-mellitus-Patienten, Eradikation von Immunschwäche-Viren in Mensch und Tier, Behandlung von Begleitinfektionen in AIDS-Patienten, Behandlung von Entzündungen viraler Genese des Respirationstraktes (Larynxpapillome, Hyperplasien, Rhinitis, Pharyngitis, Bronchitis, Pneumonien), der Sinnesorgane (Keratokonjunktivitis), des Nervensystems (Poliomyelitis, Meningoenzephalitis, Enzephalitis, subakute sklerosierende Panenzephalitis, SSPE, progressive multifokale Leukoenzephalopathie, Lymphozytäre Choriomeningitis), des Magen-Darm-Traktes (Stomatitis, Gingivostomatitis, Ösophagitis, Gastritis, Gastroenteritis, Durchfallerkrankungen), der Leber und des Gallensystems (Hepatitis, Cholangitis, hepatozelluläres Karzinom), des lymphatischen Gewebes (Mononukleose, Lymphadenitis), des hämatopoetischen Systems, der Geschlechtsorgane (Mumpsorchitis), der Haut (Warzen, Dermatitis, Herpes labialis, Fieberbläschen, Herpes Zoster, Gürtelrose), der Schleimhäute (Papillome, Konjunktivapapillome, Hyperplasien, Dysplasien), des Herz-Blutgefäß-Systems (Arteriitis, Myokarditis, Endokarditis, Perikarditis), des Nieren-Harnweg-Systems, der Geschlechtsorgane (Anogenitale Läsionen, Warzen, Genitalwarzen, spitzen Kondylome, Dysplasien, Papillome, Zervixdysplasien, Condylomata acuminata, Epidermodysplasia verruciformis), der Bewegungsorgane (Myositis, Myalgien), Behandlung der Maul- und Klauenseuche der Paarhufer, des Colorado-Zeckenfiebers, des Dengue-Syndroms, des hämorrhagisches Fiebers, der Frühsommermeningoenzephalitis (FSME) und des Gelbfiebers.

Die beschriebenen Verbindungen, d.h. die phosphororganische Verbindungen nach Formel (I) und (III) und Ester und Amide derselben an der Phosphinogruppe sowie Salze derselben zeigen eine starke zytotoxische Wirksamkeit gegenüber ein- und mehrzelligen Parasiten, insbesondere gegenüber den Erregern der Malaria und der Schlafkrankheit. Demgemäß sind die erfindungsgemäßen Verbindungen für die Behandlung von Infektionskrankheiten brauchbar, die durch Viren, Bakterien, Parasiten und Pilze bei Mensch und Tier verursacht werden. Die Verbindungen sind auch für den Einsatz zur Vorbeugung von Erkrankungen, die durch Viren, Bakterien, Parasiten und Pilze hervorgerufen werden, insbesondere als Malariaprophylaxe und als Schlafkrankheitsprophylaxe geeignet.

Die erfindungsgemäßen phosphororganischen Verbindungen, hierzu gehören im allgemeinen pharmazeutisch verträgliche Salze, Amide, Ester, ein Salz eines solchen Esters, oder aber Verbindungen, die bei Applikation die erfindungsgemäßen Verbindungen als Stoffwechselprodukte oder Abbauprodukte bereitstellen, auch "Prodrugs" genannt, können für die Verabreichung in irgendeiner geeigneten Weise analog zu bekannten antiinfektiös wirkenden Mitteln (gemischt mit einem nicht toxischen pharmazeutisch akzeptablen Träger) zubereitet werden.

Zu pharmazeutisch akzeptablen Salzen der Verbindungen gehören Salze, die die erfindungsgemäßen Verbindungen der Formeln (I) und (III) in ihrer protonierten Form als Ammoniumsalz anorganischer oder organischer Säuren, wie Salzsäure, Schwefelsäure, Zitronensäure, Maleinsäure, Fumarsäure, Weinsäure, p-Toluolsulfonsäure, bilden.

Pharmazeutisch besonders geeignet sind auch die Salze, die durch geeignete Auswahl von X₃ und X₄ gebildet werden, wie Natriumsalz, Kaliumsalz, Calciumsalz, Ammoniumsalz, Ethanolaminsalz, Triethylaminsalz, Dicyclohexylaminsalz und Salze einer Aminosäure wie Argininsalz, Asparaginsäuresalz, Glutaminsäuresalz.

Die Aktivität der Substanzen wird in einem Versuchssystem bestimmt. Dieses System beruht auf die Messung der Inhibition des Wachstums von Bakterien, Parasiten, Viren, Pilze oder Pflanzen in vitro. Hierzu werden zum Teil Versuchsverfahren verwendet, die dem Fachmann bekannt sind.

Zum Beispiel wird zur Bestimmung der Antimalaria Aktivität die Inhibition des Wachstums von Malaria Parasiten in Blutkulturen bestimmt.

Die Bestimmung der antibakteriellen Aktivität beruht auf Messung der Hemmung von Bakterienwachstum auf Nährböden und in Flüssigkulturen.

Die Bestimmung der antiviralen Aktivität beruht auf Inhibition der Bildung von viralen Elementen in Zellkulturen.

Die Bestimmung der fungiziden Aktivität beruht auf Inhibition des Wachstums von Pilzen auf Nährböden und in Flüssigkulturen.

Einige der Mikroorganismen, die untersucht werden sollen, können nur in Tiermodellen untersucht werden. Hier werden die entsprechenden Modelle angewendet.

Substanzen, die eine Wirksamkeit in den in vitro Meßsystemen zeigen, werden in in vivo Modellen weiter untersucht. Die antiparasitäre, antivirale, fungizide oder antibakterielle Aktivität wird in den entsprechenden Tiermodelle weiter evaluiert.

Das Screening nach herbizider Aktivität wird mittels Algensystemen und Messung der Isoprenemission von Pflanzen unter Standardbedingungen bestimmt.

Die pharmazeutisch wirksamen Mittel können in Form von pharmazeutischen Zubereitungen in Dosierungseinheiten zubereitet werden. Dies bedeutet, daß die Zubereitung in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie (a) Füllund Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel. Konservierungsstoffe sowie geruchs- und geschmacksverbessernde Zusätze, z. B. Pfefferminzöl und Eukalyptusöl und Süßmittel, z. B. Saccharin, enthalten.

Die Wirkstoffe der Formeln (I) und (III) sollen in den oben aufgeführten pharmazeutischen Zubereitungen, vorzugsweise in einer Konzentration von etwa 0,1 bis 99,5 Gew.-%, vorzugsweise von etwa 0,5 bis 95 Gew.-%, der Gesamtmischung vorhanden sein.

Die pharmazeutischen Zubereitungen können außer den Verbindungen der Formel (I) und (III) auch weitere pharmazeutische Wirkstoffe enthalten.

Die Verbindungen können mit bisher beschriebenen Substanzen mit antibakterieller, antiviraler, antimyktoischer und antiparasitärer Eigenschaften verwendet werden. Hierzu gehören insbesondere Verbindungen, die bereits in der Therapie Anwendung gefunden haben oder noch angewendet werden. Hierzu sind insbesondere geeignet Stoffe, die in der in der Roten Liste oder in Simon/Stille, Antibiotika-Therapie in Klinik und Praxis, 9.Auflage 1998 Schattauer Verlag, oder unter http:/www.customs.treas.gov/imp-exp/rulings/harmoniz/hrm129.html im Internet mitaufgeführt. Insbesondere können die Derivate mit Penicilline, Benzylpenicillin (Penicillin G), Phenoxypenicilline, Isoxazolylpenicilline, Aminopenicilline, Ampicillin, Amoxixillin, Bacampicillin, Carboxypenicillin, Ticarcillin, Temocillin, Acyalaminopenicilline, Azlocillin, Mezlocillin, Piperacillin, Apalcillin, Mecillinam, Cephalosporine, Cefazolin-Gruppe, Cefuroxim-Gruppe, Cefoxitin-Gruppe, Cefoxitin, Cefotetan, Cefmetazol, Latamoxef, Flomoxef, Cefotaxim-Guppe, Cefozidim, Ceftazidim-Gruppe, Ceftazidim, Cefpirom, Cefepim, übrige Cephalosporine, Cefsulodin, Cefoperazon, Oralcephalosporine der Cefalexin-Gruppe, Loracarbef, Cefprozil, neue Oralcephalosporine mit erweitertem Spektrum, Cefixim, Cefpodoxim-Proxetil, Cefuroxim-Axetil, Cefetamet, Cefotiam-Hexetil, Cefdinir, Ceftibuten, andere β-Lactam-Antibiotika, Carbapenem, Imipenem /Cilastatin, Meropenem, Biapenem, Aztreonam, β-Lactamase-Hemmer, Clavulansäure/Amoxicillin, Clavulansäure/Ticarcillin, Sulbactam/Ampicillin, Tazobactam/Piperacillin, Tetracycline, Oxytetracyclin, Rolitetraxyxlin, Doxycyclin, Minocyclin, Chloramphenicol, Aminoglykoside, Gentamicin, Tobramycin, Netilmicin, Amikacin, Spectinomyxin, Makrolide, Erythromycin, Clarithromycin, Roxithromycin, Azithromycin, Dirithromycin, Spiramycin, Josamycin, Lincosamide, Clindamycin, Fusidinsäure, Glykopeptid-Antibiotika, Vancomycin, Tecoplanin, Pristinamycin-Derivate, Fosfomycin, Antimikrobielle Folsäureantagonisten, Sulfonamide, Co-Trimoxazol, Trimethoprim, andere Diaminopyrimidin-Sulfonamid-Kombinationen, Nitrofurane, Nitrofurantoin, Nitrofurazon, Gyrase-Hemmer (Chinolone), Norfloxacin, Ciprofloxacin, Ofloxacin, Sparfloxacin, Enoxacin, Fleroxacin, Pefloxacin, Lomefloxacin, Bay Y3118, Nitroimidazole, antimykobakterielle Mittel, Isoniazid, Rifampicin, Rifabutin, Ethambutol, Pyrazinamid, Streptomycin, Capreomycin, Prothionamid, Terizidon, Dapson, Clofazimin, Lokalantibiotika, Bacitracin, Tyrothricin, Polymyxine, Neomycin, Kanamycin, Paromomycin, Mupirocin, antivirale Mittel, Acyclovir, Ganciclovir, Azidothymidin, Didanosin, Zalcitabin, Thiacytidin, Stavudin, Ribavirin, Idoxuridin, Trifluridin, Foscarnet, Amantadin, Interferone, Tibol-Derivate, Proteinase-Inhibitoren, Antimykotika, Polyene, Amphothericin B, Nystatin, Natamycin, Azole, Azole zur septischen Therapie, Miconazol, Ketoconazol, Itraconazol, Fluconazol, UK-109.496, Azole für lokale Anwendung, Clotrimazol, Econazol, Isoconazol, Oxiconazol, Bifonazol, Flucytosin, Griseofulvin, Ciclopiroxolamin, Tolnaftat, Naftifin, Terbinafin, Amorolfin, Antrachinone, Betulinic acid, Semianthrachinone, Xanthone, Naphtoquinone, Aryaminoalkohole, Chinin, Quinidine, Mefloquin, Halofantrin, Chloroquin, Amodiaquin, Acridin, Benzonaphthyridin, Mepacrin, Pyronaridin, Dapson, Sulfonamide, Sulfadoxin, Sulfalene, Trimethoprim, Proguanil, Chlorproguanil, Diaminopyrimidine, Pyrimethamin, Primaquin, Aminoquinoline, WR 238,605, Tetracyclin, Doxycyclin, Clindamycin, Norfloxacin, Ciprofloxacin, Ofloxacin, Artemisinin, Dihydroartemisinin, 10b artemether, Arteether, Atrtesunat, Atovaquon, Suramin, Melarsoprol, Nifurtmox, Stibogluconat-Natrium, Pentamidin, Amphotericin B, Metronidazol, clioquinol, Mebendazol, Niclosamid, Praziquantel, Pyrantel, Tiabendazol, Diethylcarbamazin, Ivermectin, Bithionol, Oxamniquin, Metrifonat, Piperazin, Embonat.

Ferner können die phosphororganischen Verbindungen in den pharmazeutischen Mitteln in Kombination mit Sulfonamid, Sulfadoxin, Artemisinin, Atovaquon, Chinin, Chloroquin, Hydroxychloroquin, Mefloquin, Halofantrin, Pyrimethamin, Armesin, Tetracycline, Doxycyclin, Proguanil, Metronidazol, Praziquantil, Niclosamid, Mebendazol, Pyrantel, Tiabendazol, Diethylcarbazin, Piperazin, Pyrivinum, Metrifonat, Oxamniquin, Bithionol oder Suramin oder mehreren dieser Substanzen vorliegen.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionslösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen. Therapie), Kollagen oder Knochenzement eingearbeitet werden.

Im allgemeinen hat es sich sowohl in der Human- als auch in der Veterinärmedizin als vorteilhaft erwiesen, den oder die Wirkstoffe der Formel (I) und (III) in Gesamtmengen von etwa 0,05 bis etwa 600, vorzugsweise 0,5 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 200, insbesondere 1 bis 60 mg/kg Körpergewicht. Es kann jedoch erforderlich sein, von den genannten Dosierungen abzuweichen, und zwar in Abhängigkeit von der Art und dem Körpergewicht des zu behandelnden Patienten, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch den Fachmann aufgrund seines Fachwissens erfolgen.

Die erfindungsgemäßen Verbindungen können in den üblichen Konzentrationen und Zubereitungen bei Tieren zusammen mit dem Futter bzw. mit Futterzubereitungen oder mit dem Trinkwasser gegeben werden.

Ferner können die erfindungsgemäßen Verbindungen hervorragend als Bakterizide, Fungizide und Herbizide bei Pflanzen eingesetzt werden.

Prinzipiell weiß der Fachmann, wie er die erfindungsgemäßen Stoffe zu synthetisieren hat. Im folgenden werden einige Beispiele für Synthesen angegeben.

### Beispiele

### Synthesewege für Verbindungen des Aussehen

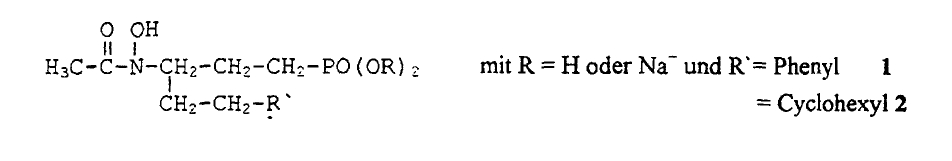

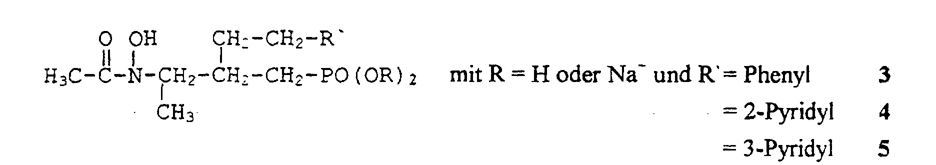

### Beispiel 1:

### 3-Oxobutylphosphonsäurediethylester (1a)

Nach Lit.: R.G. Harvey Tetrahedron 1966, 22, 2561-73 legt man in 30 ml Phenol 0,1 mol Vinylmethylketon und 0.125 mol Triethylphosphit vor und erwärmt unter Argon 24 h auf 100 °C. Die Reaktionslösung wird bis zu einer Temperatur von 110°C/8·10² Pa (6 Torr) eingeengt, wobei das verunreinigte Produkt **1a** mit in das Destillat übergeht. Die Reinigung erfolgt chromatographisch an Silicagel mit Ether/Pentan im Verhältnis 1:1 als Laufmittel.

### 3-Oxo-5-phenylpentylphosphonsäurediethylester (1b)

Zu einer gerührten Suspension von 300 mmol Natriumhydrid, die mit n-Pentan gewaschen wurde, gibt man 250 ml THF und tropft bei Zimmertemperatur 280 mmol 3-Oxobutylphosphonsäurediethylester **(1a)** zu. Nach 2-stündigem Rühren bei gleicher Temperatur wird mit einer Eis/Kochsalz-Mischung gekühlt und 1,3 Äquivalente n-BuLi zugetropft. Nach weiteren 2 h Rühren bei 0°C gibt man 310 mmol Benzylbromid - gelöst in 50 ml THF - langsam zu. Man läßt auf Zimmertemperatur erwärmen, ruhrt weitere 3 h und versetzt mit 20 ml 1 molarer HCl. Die Reaktionslösung wird auf Eiswasser gegeben, 3 x mit je 100 ml Chloroform extrahiert und die vereinigten organischen Phasen mit gesättigter Kochsalzlösung gewaschen. Die Reinigung von **1b** erfolgt chromatographisch an Silicagel mit Methylenchlorid und Essigsäureethylester als Laufmittel.
(Reaktionsbedingungen vgl.: B. Resul, J. Stjernschantz, K. No, C. Liljebris, G. Selén, M. Astin, M. Karlsson, L.Z. Bito J. Med. Chem. 1993, 36, 243-248)

### (1-Phosphonsäurediethylester)-5-phenyl-pentan-3-on-oxim (1c)

Zu einer Lösung von 18 mmol **1b** in 150 ml Ethanol gibt man 1,9 Äquivalente Hydroxylaminhydrochlorid und rührt 24 h. Dann quentscht man mit 100 ml Wasser und entfernt Ethanol und Teile von Wasser unter reduziertem Druck bis Oxim **(1c)** ausfällt. Das Produkt **(1c)** kann durch Umkristallisieren aus Ether gereinigt werden.
(Reaktionsbedingungen vgl.: T.M. Baltazor J. Org. Chem. 1980, 45, 2519-22 oder O. Tsuge et al. Bull. Chem. Soc. Jpn. 1987, 60, 2463-73)

### 3-N-(Hydroxylamino)-3-(2-phenylethyl)-propylphosphonsäure-diethylester (1d)

Natriumcyanohydridborat (NaBH₃CN) wird ohne weitere Reinigung eingesetzt. 4 mmol Oxim **1c,** gelöst in wenig Methanol, wird mit 2 Tropfen Bromcresol Grün versetzt und so lange 6 n KOH zugetropft, bis ein Farbumschlag von gelb nach grün zu beobachten ist. Man gibt 3 mmol NaBH₃CN zu, rührt 3 h bei Zimmertemperatur und quencht tropfenweise mit Methanol/HCl bis ein Farbumschlag von grün nach gelb beobachtet wird. Die Reaktionsmischung gibt man in 10 ml Wasser und stellt mit 6 n KOH einen pH-Wert > 10 ein. Nach Sättigung der wäßrigen Phase mit Kochsalz wird 5 Mal mit je 10 ml Chloroform extrahiert, diese vereinigten organischen Phasen über MgSO₄ getrocknet und das Lösungsmittel unter reduziertem Druck entfernt. Die Reinigung von Hydroxylamin (1d) erfolgt chromatographisch an Kieselgel.
(Reaktionsbedingungen vgl.: R.F. Borch, M.D. Bernstein, D.H. Dupont J. Am. Chem. Soc. 1971, 93, 2897-2904 oder A.O. Stewart et al. J. Med. Chem. 1997, 40, 1955-68)

### 3-(N-Hydroxylamino)-5-phenyl-pentylphosphonsäure (1e)

Zu 0,06 mol Ester (1d) gibt man unter Eiskühlung 130 ml konz. HCl und erwärmt 6 h unter Rückfluß (Ölbad: 150 °C). Nach dem Abkühlen wird die gelb-braun gefärbte Lösung unter reduziertem Druck eingeengt, in ca. 30 ml Wasser aufgenommen und mit Aktivkohle behandelt, bis eine nahezu farblose Lösung entsteht. Diese wird erneut unter reduziertem Druck eingeengt, mit ca. 30 ml Wasser aufgenommen und mit NaHCO₃ ein pH-Wert von 4-5 eingestellt. Der ausfallende weiß bis beige Niederschlag wird filtriert und kann mit Wasser/Ethanol gewaschen werden. 3-N-(Hydroxylamino)-5-phenyl-pentylphosphonsäure **(1e)** entsteht dabei in akzeptablen Ausbeuten und wird ohne Reinigung weiter umgesetzt.

### 3-N-Acetyl-3-N-(hydroxylamino)-5-phenyl-pentylphosphonsäure (1)

0,013 mol 3-(N-Hydroxylamino)-5-phenyl-pentylphosphonsäure (1e) wird in 20 ml Wasser vorgelegt und 4,51 g (0,044 mol) Essigsäureanhydrid bei Zimmertemperatur dazu getropft. Nach 1,5-stündigem Rühren bei gleicher Temperatur stellt man mit 2 n NaOH einen pH-Wert von 2,5 ein, engt die Lösung unter reduziertem Druck ein, nimmt in 40 ml Wasser auf und engt erneut ein. Diese Prozedur wird einmal wiederholt. Dann wäscht man mehrmals mit Ether, wobei dieser per Dekantieren entfernt wird, löst in ca. 5-10 ml Ethanol und wiederholt das Waschen mit Ether. Die wäßrige Phase wird auf 50 ml aufgefüllt und mit 2 n NaOH ein pH-Wert von 6,5 eingestellt. Nach Abziehen flüchtiger Bestandteile unter reduziertem Druck wird zum Entfernen restlichen Wassers mit n-Butanol versetzt, das ebenfalls unter reduziertem Druck (bis Ölpumpenvakuum) entfernt wird. Das zurückbleibende Öl kocht man mit Isopropanol auf, das verworfen wird und zerreibt das zurückbleibende glasartige Harz zu einem Feststoff, der aus Methanol durch Zugabe größerer Mengen Aceton umkristallisiert werden kann.

### Beispiel 2:

### 3-N-Acetyl-3-N-(hydroxylamino)-5-cyclohexyl-pentylphosphon-säure (2)

Die Synthese von **2** folgt der unter **1** beschriebenen. Die einzige Änderung tritt im zweiten Reaktionsschritt auf, wo Brommethylcyclohexan anstelle von Benzylbromid eingesetzt wird. So erhält man nicht 3-Oxo-5-phenyl-pentylphosphonsäurediethyl-ester **(1b)** sondern 3-Oxo-5-cyclohexyl-pentylphosphonsäuredi-ethylester **(2b),** das wie oben beschrieben weiter behandelt wird.

### Beispiele 3 bis 5:

Mögliche Edukte sind die an C5-Phenyl, 2-Pyridyl oder 3-Pyridyl substituierten Pentan-2-one, die in der Literatur bereits beschrieben sind:

### 5-Phenyl-2-pentanon (3a)

Nach Lit.: F.C. Montgomery, W.H. Saunders, Jr. J. Org. Chem. 1976, 41, 2368-72 wird zu 200 ml absolutem Ethanol 7,6 g Natrium gegeben. Nachdem sich dieses gelöst hat, tropft man 43 g (0,33 mol) Acetylessigsaureethylester innerhalb einer Stunde zu, erhitzt eine Stunde unter Rückfluß, läßt 65 g 2-Phenylethylbromid langsam dazu tropfen und erhitzt erneut unter Rückfluß (21 h). Nach Abkühlen und Filtrieren wird destilliert. Das Produkt wird zunächst mit 350 ml 5 %igem Natriumhydroxyd 5 h auf 90 °C erwärmt, dann mit 150 ml 50 %iger Schwefelsäure 5 h bei gleicher Temperatur gerührt. Nach dem Abkühlen wird das Produkt 48 h lang stehen gelassen, mit Diethylether extrahiert, die vereinigten Extrakte über Natriumsulfat getrocknet und **3a** schließlich destillativ in guter Ausbeute gewonnen (Sdp.: 132-134 °C, 2,3·10³ Pa(17 Torr)).

### 5-(2-Pyridyl)-2-pentanon (4a)

Nach Lit.: F. Noel, Albertson J. Am. Chem. Soc. 1950, 72, 2594-99 wird eine Reaktionsmischung aus 50 g Acetylaceton, 1,5 g Natrium und 108 g 2-Vinylpyridin 7 h unter Rückfluß erhitzt. Die Produkte lassen sich bei 1,5·10² Pa (1,1 Torr) als gelbes Öl destillieren (Sdp.: 90 - 127 °C). Fraktionierte Destillation dieses Öls ergibt 11,9 g 5-(2-Pyridyl)-2-pentanon **(4a)** vom Siedepunkt 88 - 105 °C und 1,3·10² Pa (1,0 Torr).

### 5-(3-Pyridyl)-2-pentanon (5a):

(nach: J.W. Tilley, P. Levitan, J. Lind, A.F. Welton, H.J. Crowley, L.D. Tobias, M. O'Donnell J. Med. Chem. 1987, 30, 185-93; Swern-Oxidation des Alkohols)

59,5 ml Oxalylchlorid in 1,4 1 absolutem Dichlormethan werden auf -60 °C gekühlt und 96 ml absolutes DMSO - gelöst in 280 ml absolutes Dichlormethan - langsam zugetropft. Nach 15-minütigem Rühren bei -60 °C tropft man 93 g 5-(3-Pyridyl)-2-pentanol in 470 ml des gleichen Lösungsmittels bei -60 °C zu. Nach 20-minütigem Rühren gibt man 396 ml Triethylamin langsam hinzu, rührt für weitere 20 min bei -60 °C und läßt auf Zimmertemperatur erwärmen. Schließlich gibt man die Reaktionslösung auf 700 ml Eiswasser, das zuvor mit NaOH-Pastillen versetzt wurde. Nach dem Trennen der Phasen wird die wäßrige, alkalische Phase 3x mit je 300 ml Dichlormethan extrahiert, die vereinigten organischen Phasen mit 4 x 500 ml Wasser gewaschen, über Kaliumcarbonat getrocknet und eingeengt. Nach Destillation erhält man in guter Ausbeute **5a** vom Sdp.: 98-104°C, 3·10¹ Pa (0,2 Torr).

### Brommethylphosphonsäurediethylester (3b)

(nach P.C. Crofts, G.M. Kosolapoff J. Am. Chem. Soc. 1953, 75, 5738-40)

126 g Triethylphosphit und 162 g Dibrommethan werden in einem Autoklaven 4 h auf 170 °C erhitzt. Aus der Reaktionsmischung läßt sich Brommethylphosphonsäurediethylester in mäßiger Ausbeute durch Destillation isolieren (Sdp.: ca. 50 °C, 7 Pa (0,05 Torr)). Eine zweite fraktionierte Destillation wurde angeschlossen.

### Beispiele 7 bis 9:

Die Darstellung der 3-Oxo-2-(2-R'-ethyl)-butylphosphonsäure-diethylester mit R' = Phenyl, 2-Pyridyl und 3-Pyridyl erfolgt durch Erzeugen des thermodynamisch stabilisierten Trimethylsilylenolethers der Ketone **3a, 4a** und **5a** und nachfolgende Umsetzung mit Brommethylphosphonsäurediethylester **(3b):**

### 3-Oxo-2-(2-phenylethyl)-butylphosphonsäurediethylester (3c)

(Exemplarische Umsetzung von **3a** mit Brommethylphosphonsäurediethylester. Diese über den thermodynamisch stabilisierten Trimethylsilylenolether verlaufende Reaktion läßt sich auf gleiche Weise mit **4a** und **5a** durchführen)

### Darstellung der Trimethylsilylenolether; hier exemplarisch: 5-Phenyl-2-Trimethylsilyl-2-pentanol (3c')

Zu 0,3 mol Trimethylchlorsilan und 0,6 mol Triethylamin in 100 ml absolutem Dimethylformamid (DMF) tropft man 0,25 mol 5-Phenyl-2-pentanon (3a) und rührt 3 Tage bei einer Temperatur von 110 °C. Nach Verdünnen der Lösung mit 200 ml Pentan wäscht man 3 mal mit je 300 ml NaHCO₃-haltiger kalter wäßriger Lösung. Die organische Phase wird nun nacheinander mit kalter 1,5 molarer wäßriger HCl und mit kalter wäßriger NaHCO₃ Läsung gewaschen, wobei schnell gearbeitet werden muß. Nach Trocknen und Einengen unter reduziertem Druck bis zu maximalem Ölpumpenvakuum können Nebenprodukte und Edukte entfernt werden. Die Isolierung des thermodynamisch stabilisierten Enolethers **3c'** gelingt per Hochdruckflüssigkeitschromatographie an einer Diolphase (UV-Detektion, Laufmittel: Dichlormethan, *tert*-Butylmethylether).
(Reaktionsbedingungen vgl.: H.O. House, L.J. Czuba, M. Gall, H.D. Olmstead J. Org. Chem. 1969, 34, 2324 und I. Peterson Tetrahedron 1988, 44, 4207-19 sowie I. Fleming, I. Paterson Synthesis, 1979, 736-38.

### Darstellung der 2-(2-R'-ethyl)-substituierten 3-Oxo-butylphos-phonsäureester; hier exemplarisch: 3-Oxo-2-(2-phenylethyl)-butylphosphonsäurediethylester (3c)

5,5 mmol TiCl₄ in 5 ml absolutem Dichlormethan wird bei -20 °C unter Argon zu einer Lösung von 5 mmol O-silyliertem Enolat **3c'** und 6 mmol Brommethylphosphonsäurediethylester **(3b)** in 5 ml absolutem Dichlormethan getropft. Nach ca. einer Stunde gibt man die Reaktionslösung auf 25 ml wäßrige gesättigte NaHCO₃-Lösung, extrahiert mehrmals mit Ether, trocknet die vereinigten organischen Phasen über MgSO₄, engt unter reduziertem Druck ein und reinigt das Produkt **3c** per Säulenchromatographie an Silicagel.

3-Oxo-2-(2-phenylethyl)-butylphosphonsäurediethylester **(3c)** sowie 3-Oxo-2-[2-(2-pyridyl)ethyl]-butylphosphon-säurediethyl-ester **(4c)** und 3-Oxo-2-[2-(3-pyridyl)ethyl]-butylphosphon-säurediethylester **(5c)** lassen sich dann, wie bereits unter **1** beschrieben, durch Überführung ins Oxim, die Reduktion zum Hydroxylamin, die sich anschießende Hydrolyse und eine Acetylierung in die Produkte
3-Acetyl-3-(N-hydroxylamino)-3-methyl-2-(2-phenylethyl)-propylphosphonsäure **(3)** 3-Acetyl-3-(N-hydroxylamino)-3-methyl-2-[2-(2-pyridyl)ethyl]-propylphosphonsäure **(4)** und 3-Acetyl-3-(N-hydroxylamino)-3-methyl-[2-(3-pyridyl)ethyl]-propylphosphonsäure **(5)** überführen. Die Reaktionsbedingungen samt eingesetzter Relationen entsprechen den unter **Beispiel 1** beschriebenen.

### Beispiel 10

### Antibakterielle Wirkung der oben angegebenen Verbindungen 1 bis 5

In 5 Kulturröhrchen wurde eine Verdünnungsserie mit den Konzentrationen 500, 100, 50, 10 und 0 µmol l⁻¹ der einzelnen Verbindungen **1** bis **5** in LB-Medium in einem Volumen von 0,5 ml vorgelegt. Die Röhrchen wurden mit je 10 µl aus der Übernachtkultur von E. coli K12 inokuliert und über Nacht bei 37°C geschüttelt. Das Wachstum der Bakterien wurde durch Trübung des Mediums beurteilt. Die Ergebnisse sind in Tabelle 1 aufgerührt.

### Beispiel 11

Die Antimalaria-Wirksamkeit der Verbindungen 1 bis 5 wurde an in-vitro-Kulturen des Malaria-Erregers Plasmodium falciparum bestimmt. Die Vertiefungen einer 96-well- Mikrotiterplatte wurden mit je 200 µl einer asynchronen Plasmodium falciparum-Kultur bei 0,4 % Parasitämie und 2 % Hämatokrit beschickt. Dann wurde eine serielle Verdünnungsreihe der Verbindungen in Dreierschritten zwischen Konzentrationen von 100 bis 0,14 µmol l⁻¹ hergestellt. Die Platten werden bei 37°C, 3 % CO₂ und 5 % O₂ über einen Zeitraum von 48 Stunden inkubiert. Dann wurden zu jedem well 30 µl Medium supplementiert mit 27 µCi ml⁻¹ [³H]-Hypoxanthin zugefügt. Nach 24-stündiger Inkubation wurden die Parasiten durch Filtration auf Glasfaserfilter geerntet und die incorporierte Radioaktivität gemessen. Die Inhibition des Parasitenwachstums wurde als prozentuale Inhibition der Tritiumcorporation gemessen. Die Inhibition des Parasitenwachstums wurde als prozentuale Inhibition der Tritiumcorporation bezogen auf einen Vergleich ohne Substanz ausgedrückt. Durch Extrapolation der Werte wurde die halbmaximale inhibitorische Konzentration (IC50) der Substanz bestimmt. Die Ergebnisse sind in Tabelle 1 aufgerührt.

**Tabelle 1**

| | Beispiel 10 | Beispiel 11 |
|---|---|---|
| Verbindungen | IC50-Werte E. coli (nM) | IC50-Werte Plasmodium falciparum (nM) |
| **1** | 468 | 215 |
| **2** | 324 | 157 |
| **3** | 762 | 405 |
| **4** | 736 | 375 |
| **5** | 318 | 183 |

## Patentansprüche

1. Phosphororganische Verbindungen der allgemeinen Formel (I) 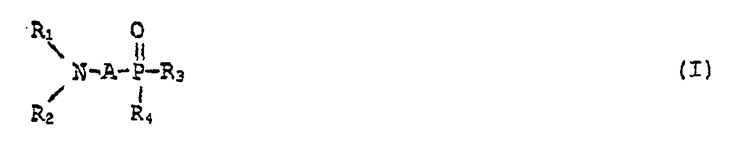 in der R₁ und R₂ gleich oder verschieden sind und aus der Gruppe ausgewählt sind, die aus Wasserstoff, substituiertem und unsubstituiertem C₁₋₉-Alkyl, substituiertem und unsubstituiertem Hydroxy-C₁₋₉-alkyl, substituiertem und unsubstituiertem C₁₋₉-Alkenyl, substituiertem und unsubstituiertem C₁₋₉-Alkinyl, substituiertem und unsubstituiertem Aryl, substituiertem und unsubstituiertem Acyl, substituiertem und unsubstituiertem Cycloalkyl, substituiertem und unsubstituiertem Aralkyl, substituiertem und unsubstituiertem heterocyclischen Rest, Halogen, OX₁ und OX₂ besteht,
wobei X₁ und X₂ gleich oder verschieden sein können und aus der Gruppe ausgewählt sind, die aus Wasserstoff, substituiertem und unsubstituiertem C₁₋₉-Alkyl, substituiertem und unsubstituiertem Hydroxy-C₁₋₉-alkyl, substituiertem und unsubstituiertem C₁₋₉-Alkenyl, substituiertem und unsubstituiertem C₁₋₉-Alkinyl, substituiertem und unsubstituiertem Aryl, substituiertem und unsubstituiertem Acyl, substituiertem und unsubstituiertem Cycloalkyl, substituiertem und unsubstituiertem Aralkyl, substituiertem und unsubstituiertem heterocyclischen Rest besteht,
in der A der folgenden Formel (II) entspricht: 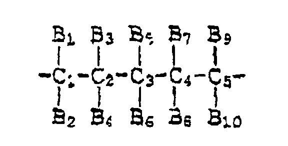 wobei ein oder mehrere der Kohlenstoffatome, ausgewählt aus der Gruppe C₃, C₄, C₅, mitsamt ihren Substituenten auch wegfallen können, und mindestens ein vorliegender Substituent von B₁ bis B₁₀ eine C₃₋₈-Cycloalkyl-(C₀₋₉)-alkylgruppe ist, wobei sowohl die C₃₋₈-Cycloalkylgruppe als auch die C₀₋₉-Alkylgruppe ein oder mehrere Doppelbindungen aufweisen können und ein oder zwei Kohlenstoffatome der Cycloalkylgruppe durch Stickstoff-, Sauerstoff- oder Schwefelatome ersetzt sein können, und wobei sowohl die Cycloalkylgruppe als auch die Alkylgruppe mit Hydroxy-, Halogen-, Amino-, Oxogruppen mit verzweigten oder unverzweigten C₁₋₉-Alkylgruppen und C₂₋₉-Alkenylgruppen substituiert sein können, wobei die C₁₋₉-Alkylgruppen und C₂₋₉-Alkenylgruppen mit Wasserstoff-, Hydroxy-, Amino-, Halogen- und Oxogruppen substituiert sein können, und die restlichen vorliegenden Substituenten B₁ bis B₁₀ aus der Gruppe ausgewählt sind, die aus Wasserstoff, Hydroxy-, Halogen-, Aminogruppen, C₁-₂₆-Alkylresten, C₁₋₂₆-Alkoxy-resten, C₁₋₂₆-Alkoxy-C₁₋₂₆-Alkylresten besteht oder beide Substituenten eines C-Aroms zusammen eine Oxogruppe bilden, wobei jeder C₁₋₂₆-Alkylrest und jeder C₁₋₂₆-Alkoxyrest verzweigt oder unverzweigt und gesättigt oder mit ein oder mehreren Doppelbindungen ungesätrigt sein kann und mit Hydroxy-, Amino-, Halogen- und Oxogruppen substituiert sein kann,
in der R₃ und R₄ gleich oder verschieden sind und aus der Gruppe ausgewählt ist, die aus substituiertem und unsubstituiertem C₁₋₂₆-Alkyl, Hydroxy-C₁₋₂₆-alkyl, substituiertem und unsubstituiertem Aryl, substituiertem und unsubstituiertem Acyl, substituiertem und unsubstituiertem Aralkyl, substituiertem und unsubstituiertem C₁₋₂₆-Alkenyl, substituiertem, und unsubstituiertem C₁₋₂₆-Alkinyl, substituiertem und unsubstituiertem Cycloalkyl, substituiertem und unsubstituiertem heterocyclischem Rest, Halogen, OX₃ und OX₄ besteht,
wobei X₃ und X₄ gleich oder verschieden sind und aus Wasserstoff, substituiertem und unsubstituiertem C₁₋₂₆-Alkyl, substituiertem und unsubstituiertem Hydroxy- C₁₋₂₆-alkyl, substituiertem und unsubstituiertem Aryl, substituiertem und unsubstituiertem Aralkyl, substituiertem und unsubstituiertem C₁₋₂₆-Alkenyl, substituiertem und unsubstituiertem C₁₋₂₆-Alkinyl, substituiertem und unsubstituiertem Cycloalkyl, substituiertem und unsubstituiertem heterocyclischem Rest, einem Silyl, einem Kation einer organischen und anorganischen Base, insbesondere einem Metall der ersten, zweiten oder dritten Hauptgruppe des Periodensystems, Ammonium, substituiertem Ammonium und Ammoniumverbindungen, die sich von Ethylendiamin oder Aminosäuren ableiten, besteht, und deren pharmazeutisch akzeptablen Salze, Ester und Salze der Ester.

2. Verbindung nach Anspruch 1, **dadurch gekennzeichnet, daß** die phosphororganischen Verbindungen der Formel (II) 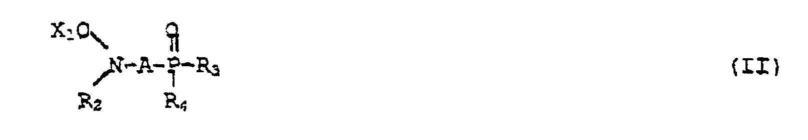 entsprechen, wobei
X₁ Wasserstoff ist und R₂ ein Acylrest, besonders bevorzugt ein Formylrest oder Acetylrest, ist und R₃, R₄ und A die gleiche Bedeutung wie in Formel (I) haben.

3. Verbindung nach Anspruch 1 oder Anspruch 2, **dadurch gekennzeichnet, daß** X₃ und X₄ aus der Gruppe ausgewählt sind, die aus OX₃ und OX₄ besteht, und X₃ und X₄ aus der Gruppe ausgewählt sind, die aus Wasserstoff, einem Metall der ersten, zweiten oder dritten Hauptgruppe des Periodensystems, Ammonium, substituiertem Ammonium, oder Ammoniumverbindungen, die sich von Ethylendiamin oder Aminosäuren ableiten.

4. Verbindung nach einem der vorhergehenden Ansprüche, **dadurch gekennzeichnet, daß** die Kohlenstoffkette von A mit der Formel (II) aus drei Kohlenstoffatomen C₁, C₂, C₃ besteht.

5. Verbindung nach einem der Ansprüche 1 bis 3, **dadurch gekennzeichnet, daß** B₁ und B₂ zusammen oder B₇ und B₈ zusammen eine Oxogruppe bilden und die Kohlenstoffkette in A aus den vier Kohlenstoffatomen C₁, C₂, C₃, C₄ besteht.

6. Verbindung nach einem der Ansprüche 1 bis 3, **dadurch gekennzeichnet, daß** die Kohlenstoffkette von A mit der Formel (II) aus vier Kohlenstoffatomen C₁, C₂, C₃, C₄ besteht und B₇ oder B₈ oder beide eine Hydroxygruppe sind.

7. Verbindung nach einem der Ansprüche 1 bis 3, **dadurch gekennzeichnet, daß** die Kohlenstoffkette bevorzugt aus den 5 Kohlenstoffatomen C₁, C₂, C₃, C₄, C₅ besteht, wobei B₁ und B₂ zusammen eine Oxogruppe bilden und B₉ oder B₁₀ eine Hydroxylgruppe sind oder B₉ und B₁₀ zusammen ebenfalls eine Oxogruppe bilden.

8. Verbindung nach Anspruch 6 oder Anspruch 7 in Abhängigkeit von Anspruch 1 oder 2, **dadurch gekennzeichnet, daß** R₃ oder R₄ oder beide Methylengruppen sind.

9. Verwendung von phosphororganischen Verbindungen nach einem der Ansprüche 1 bis 8 zur Herstellung eines Arzneimittels für die Behandlung von infektiösen Prozessen bei Mensch und Tier, die durch Viren, Bakterien, Pilze oder Parasiten hervorgerufen werden und als Fungizid, Bakterizid oder Herbizid bei Pflanzen.

10. Verwendung nach Anspruch 9 zur Herstellung eines Arzneimittels für die Behandlung von Infektionen, verursacht durch Bakterien, Viren, Pilze oder ein- oder mehrzellige Parasiten.

11. Verwendung nach Anspruch 10 zur Herstellung eines Arzneimittels für die Behandlung von Infektionen, die durch Bakterien hervorgerufen werden, die aus der Gruppe ausgewählt sind, die aus Bakterien der Familie Propionibacteriaceae, insbesondere der Gattung Propionibacterium, insbesondere die Art Propionibacterium acnes, Bakterien der Familie Actinomycetaceae, insbesondere der Gattung Actinomyces, Bakterien der Gattung Corynebacterium, insbesondere die Arten Corynebacterium diphteriae und Corynebacterium pseudotuberculosis, Bakterien der Familie Mycobacteriaceae, der Gattung Mycobacterium, insbesondere die Arten Mycobacterium leprae, Mycobacterium tuberculosis, Mycobacterium bovis und Mycobacterium avium, Bakterien der Familie Chlamydiaceae, insbesondere die Spezies Chlamydia trachomatis und Chlamydia psittaci, Bakterien der Gattung Listeria, insbesondere die Art Listeria monocytogenes, Bakterien der Art Erysipelthrix rhusiopathiae, Bakterien der Gattung Clostridium, Bakterien der Gattung Yersinia, der Spezies Yersinia pestis, Yersinia pseudotuberculosis, Yersinia enterocolitica und Yersinia ruckeri, Bakterien der Familie Mycoplasmataceae, der Gattungen Mycoplasma und Ureaplasma, insbesondere die Art Mycoplasma pneumoniae, Bakterien der Gattung Brucella, Bakterien der Gattung Bordetella, Bakterien der Familie Neisseriaceae, insbesondere der Gattungen Neisseria und Morexella, insbesondere die Arten Neisseria meningitides, Neisseria gonorrhoeae und Moraxella bovis, Bakterien der Familie Vibrionaceae, insbesondere der Gattungen Vibrio, Aeromonas, Plesiomonas und Photobacterium, insbesondere die Arten Vibrio cholerae, Vibrio anguillarum und Aeromonas salmonicidas, Bakterien der Gattung Campylobacter, insbesondere die Arten Campylobacter jejuni, Campylobacter coli und Campylobacter fetus, Bakterien der Gattung Helicobacter, insbesondere die Art Helicobacter pylori, Bakterien der Familien Spirochaetaceae und der Leptospiraceae, insbesondere der Gattungen Treponema, Borrelia und Leptospira, insbesondere Borrelia burgdorferi, Bakterien der Gattung Actinobacillus, Bakterien der Familie Legionellaceae, der Gattung Legionella, Bakterien der Familie Rickettsiaceae und Familie Bartonellaceae, Bakterien der Gattungen Nocardia und Rhodococcus, Bakterien der Gattung Dermatophilus, Bakterien der Familie Pseudomonadaceae, insbesondere der Gattungen Pseudomonas und Xanthomonas, Bakterien der Familie Enterobacteriaceae, insbesondere der Gattungen Escherichia, Klebsiella, Proteus, Providencia, Salmonella, Serratia und Shigella, Bakterien der Familie Pasteurellaceae, insbesondere der Gattung Haemophilus, Bakterien der Familie Micrococcaceae, insbesondere der Gattungen Micrococcus und Staphylococcus, Bakterien der Familie Streptococcaceae, insbesondere der Gattungen Streptococcus und Enterococcus und Bakterien der Familie Bacillaceae, insbesondere der Gattungen Bacillus und Clostridium besteht, und bei der Helicobacter-Eradikationstherapie bei Ulcera des Magendarmtraktes.

12. Verwendung nach Anspruch 10 zur Herstellung eines Arzneimittels für die Behandlung von Infektionen, die durch Viren hervorgerufen werden, die aus der Gruppe ausgewählt sind, die aus Viren der Gattung Parvoviridae, insbesondere Parvoviren, Dependoviren, Densoviren, Viren der Gattung Adenoviridae, insbesondere Adenoviren, Mastadenoviren, Aviadenoviren, Viren der Gattung Papovaviridae, insbesondere Papovaviren, insbesondere Papillomaviren (sogenannte Warzenviren), Polyomaviren, insbesondere JC-Virus, BK-Virus, und Miopapovaviren, Viren der Gattung Herpesviridae, insbesondere Herpes-Simplex-Viren, der Varizellen/Zoster-Viren, menschlicher Zytomegalievirus, Epstein-Barr-Viren, humanes Herpesvirus 6, humanes Herpesvirus 7, humanes Herpesvirus 8, Viren der Gattung Poxviridae, insbesondere Pockenviren, Orthopox- Parapox-, Molluscum-Contagiosum-Virus, Aviviren, Capriviren, Leporipoxviren, primär hepatotropen Viren, insbesondere Hepatitisviren, wie Hepatitis-A-Viren, Hepatitis-B-Viren, Hepatitis-C-Viren, Hepatitis-D-Viren, Hepatitis-E-Viren, Hepatitis-F-Viren, Hepatits-G-Viren, Hepadnaviren, insbesondere sämtliche Hepatitisviren, wie Hepatitis-B-Virus, Hepatitis-D-Viren, Viren der Gattung Picornaviridae, insbeondere Picornaviren, alle Enteroviren, alle Polioviren, alle Coxsackieviren, alle Echoviren, alle Rhinoviren, Hepatitis-A-Virus, Aphthoviren, Viren der Gattung Calciviridae, insbesondere Hepatitis-E-Viren, Viren der Gattung Reoviridae, insbesondere Reoviren, Orbiviren, Rotaviren, Viren der Gattung Togaviridae, insbesondere Togaviren, Alphaviren, Rubiviren, Pestiviren, Rubellavirus, Viren der Gattung Flaviviridae, insbesondere Flaviviren, FSME-Virus, Hepatitis-C-Virus, Viren der Gattung Orthomyxoviridae, insbesondere Influenzaviren, Viren der Gattung Paramyoxviridae, insbesondere Paramyxoviren, Morbillivirus, Pneumovirus, Masernvirus, Mumpsvirus, Viren der Gattung Rhabdoviridae, insbesondere Rhabdoviren, Rabiesvirus, Lyssavirus, viskuläres Stomatitisvirus, Viren der Gattung Coronaviridae, insbesondere Coronaviren, Viren der Gattung Bunyaviridae, insbesondere Bunyaviren, Nairovirus, Phlebovirus, Uukuvirus, Hantavirus, Hantaanvirus, Viren der Gattung Arenaviridae, insbesondere Arenaviren, lymphozytäres Choriomeningitis-Virus, Viren der Gattung Retroviridae, insbesondere Retroviren, alle HTL-Viren, humanes T-cell Leukämievirus, Oncornaviren, Spumaviren, Lentiviren, alle HI-Viren, Viren der Gattung Filoviridae, insbesondere Marburg- und Ebolavirus, Slow-Viren, Prionen, Onkoviren und Leukämieviren besteht.

13. Verwendung nach Anspruch 10 zur Herstellung eines Arzneimittels für die Vorbeugung und Behandlung von Infektionen verursacht durch einzellige Parasiten, nämlich Erreger der Malaria, der Schlafkrankheit, der Chagas-Krankheit, der Toxoplasmose, der Amöbenruhr, der Leishmaniosen, des Trichomoniasis, der Pneumozystose, der Balantidiose, der Kryptosporidiose, der Sarkozystose, der Akantha-möbose, der Naeglerose, der Kokzidiose, der Giardiose und der Lambliose.

## Claims

1. Organophosphorus compounds of the general formula (I) 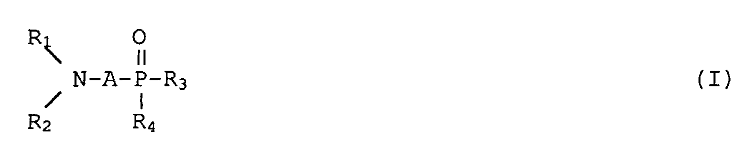 in which R₁ and R₂ are identical or different and are selected from the group which consists of hydrogen, substituted and unsubstituted C₁₋₉-alkyl, substituted and unsubstituted hydroxy-C₁₋₉-alkyl, substituted and unsubstituted C₁₋₉-alkenyl, substituted and C₁₋₉-alkynyl, substituted and unsubstituted aryl, substituted and unsubstituted acyl, substituted and unsubstituted cycloalkyl, substituted and unsubstituted aralkyl, substituted and unsubstituted heterocyclic residue, halogen, OX₁ and OX₂,
wherein X₁ and X₂ may be identical or different and are selected from the group which consists of hydrogen, substituted and unsubstituted C₁₋₉-alkyl, substituted and unsubstituted hydroxy-C₁₋₉-alkyl, substituted and unsubstituted C₁₋₉-alkenyl, substituted and unsubstituted C₁₋₉-alkynyl, substituted and unsubstituted aryl, substituted and unsubstituted acyl, substituted and unsubstituted cycloalkyl, substituted and unsubstituted aralkyl, substituted and unsubstituted heterocyclic residue,
in which A is of the following formula (II): 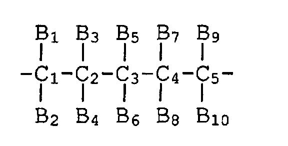 wherein one or more of the carbon atoms selected from the group C₃, C₄, C₅ together with their substituents may also be absent, and at least one substituent of B₁ to B₁₀ present is a C₃₋₈-cycloalkyl-(C₀₋₉-)-alkyl group, wherein both the C₃₋₈-cycloalkyl group and the C₀₋₉-alkyl group may comprise one or more double bonds and one or two carbon atoms of the cycloalkyl group may be replaced by nitrogen, oxygen or sulphur atoms, and wherein both the cycloalkyl group and the alkyl group may be substituted with hydroxy, halogen, amino, oxo groups with branched or unbranched C₁₋₉-alkyl groups and C₂₋₉-alkenyl groups, wherein the C₁₋₉-alkyl groups and C₂₋₉-alkenyl groups may be substituted with hydrogen, hydroxy, amino, halogen and oxo groups, and the remaining substituents B₁ to B₁₀ present are selected from the group which consists of hydrogen, hydroxy, halogen, amino groups, C₁₋₂₆-alkyl residues, C₁₋₂₆-alkoxy residues, C₁₋₂₆-alkoxy-C₁₋₂₆-alkyl residues or both substituents of a C-atom together form an oxo group, wherein each C₁₋₂₆-alkyl residue and each C₁₋₂₆-alkoxy residue may be branched or unbranched and be saturated or unsaturated with one or more double bonds and may be substituted with hydroxy, amino, halogen and oxo groups,
in which R₃ and R₄ are identical or different and are selected from the group which consists of substituted and unsubstituted C₁₋₂₆-alkyl, hydroxy-C₁₋₂₆-alkyl, substituted and unsubstituted aryl, substituted and unsubstituted acyl, substituted and unsubstituted aralkyl, substituted and unsubstituted C₁₋₂₆-alkenyl, substituted and unsubstituted C₁₋₂₆-alkynyl, substituted and unsubstituted cycloalkyl, substituted and unsubstituted heterocyclic residue, halogen, OX₃ and OX₄,
wherein X₃ and X₄ are identical or different and consist of hydrogen, substituted and unsubstituted C₁₋₂₆-alkyl, substituted and unsubstituted hydroxy-C₁₋₂₆-alkyl, substituted and unsubstituted aryl, substituted and unsubstituted aralkyl, substituted and unsubstituted C₁₋₂₆-alkenyl, substituted and unsubstituted C₁₋₂₆-alkynyl, substituted and unsubstituted cycloalkyl, substituted and unsubstituted heterocyclic residue, a silyl, a cation of an organic and inorganic base, in particular a metal of main groups I, II or III of the periodic system, ammonium, substituted ammonium and ammonium compounds derived from ethylenediamine or amino acids, and the pharmaceutically acceptable salts, esters thereof and salts of the esters.

2. Compound according to claim 1, **characterised in that** the organophosphorus compounds are of the formula (II) 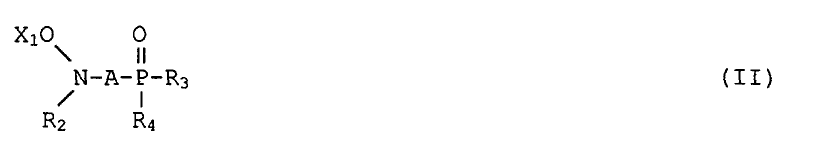 wherein X₁ is hydrogen and R₂ an acyl residue, particularly preferably a formyl residue or acetyl residue, and R₃, R₄ and A have the same meaning as in formula (I).

3. Compound according to claim 1 or claim 2, **characterised in that** X₃ and X₄ are selected from the group which consists of OX₃ and OX₄, and X₃ and X₄ are selected from the group which of hydrogen, a metal of main groups I, II or III of the periodic system, ammonium, substituted ammonium, or ammonium compounds derived from ethylenediamine or amino acids.

4. Compound according to one of the preceding claims, **characterised in that** the carbon chain of A with the formula (II) consists of three carbon atoms C₁, C₂, C₃.

5. Compound according to one of claims 1 to 3, **characterised in that** B₁ and B₂ together or B₇ and B₈ together form an oxo group and the carbon chain in A consists of four carbon atoms C₁, C₂, C₃, C₄.

6. Compound according to one of claims 1 to 3, **characterised in that** the carbon chain of A with the formula (II) consists of four carbon atoms C₁, C₂, C₃, C₄ and B₇ or B₈ or both are a hydroxy group.

7. Compound according to one of claims 1 to 3, **characterised in that** the carbon chain preferably consists of 5 carbon atoms C₁, C₂, C₃, C₄, C₅, wherein B₁ and B₂ together form an oxo group and B₉ or B₁₀ are a hydroxyl group or B₉ and B₁₀ together also form an oxo group.

8. Compound according to claim 6 or claim 7 dependent upon claim 1 or 2, **characterised in that** R₃ or R₄ or both are methylene groups.

9. Use of organophosphorus compounds according to one of claims 1 to 8 for preparing a pharmaceutical preparation for the treatment of infectious processes in humans and animals which are caused by viruses, bacteria, fungi or parasites and as a fungicide, bactericide or herbicide in plants.

10. Use according to claim 9 for the treatment of infections caused by bacteria, viruses, fungi or uni- or multicellular parasites.

11. Use according to claim 10 for preparing a pharmaceutical preparation for the treatment of infections which are caused by bacteria which are selected from the group which consists of bacteria of the family Propionibacteriaceae, in particular of the genus Propionibacterium, in particular the species Propionibacterium acnes, bacteria of the family Actinomycetaceae, in particular of the genus Actinomyces, bacteria of the genus Corynebacterium, in particular the species Corynebacterium diphtheriae and Corynebacterium pseudotuberculosis, bacteria of the family Mycobacteriaceae, of the genus Mycobacterium, in particular the species Mycobacterium leprae, Mycobacterium tuberculosis, Mycobacterium bovis and Mycobacterium avium, bacteria of the family Chlamydiaceae, in particular the species Chlamydia trachomatis and Chlamydia psittaci, bacteria of the genus Listeria, in particular the species Listeria monocytogenes, bacteria of the species Erysipelthrix rhusiopathiae, bacteria of the genus Clostridium, bacteria of the genus Yersinia, the species Yersinia pestis, Yersinia pseudotuberculosis, Yersinia enterocolitica and Yersinia ruckeri, bacteria of the family Mycoplasmataceae, of the genera Mycoplasma and Ureaplasma, in particular the species Mycoplasma pneumoniae, bacteria of the genus Brucella, bacteria of the genus Bordetella, bacteria of the family Neisseriaceae, in particular of the genera Neisseria and Moraxella, in particular the species Neisseria meningitides, Neisseria gonorrhoeae and Moraxella bovis, bacteria of the family Vibrionaceae, in particular of the genera Vibrio, Aeromonas, Plesiomonas and Photobacterium, in particular the species Vibrio cholerae, Vibrio anguillarum and Aeromonas salmonicidas, bacteria of the genus Campylobacter, in particular the species Campylobacter jejuni, Campylobacter coli and Campylobacter fetus, bacteria of the genus Helicobacter, in particular the species Helicobacter pylori, bacteria of the families Spirochaetaceae and Leptospiraceae, in particular of the genera Treponema, Borrelia and Leptospira, in particular Borrelia burgdorferi, bacteria of the genus Actinobacillus, bacteria of the family Legionellaceae, of the genus Legionella, bacteria of the family Rickettsiaceae and family Bartonellaceae, bacteria of the genera Nocardia and Rhodococcus, bacteria of the genus Dermatophilus, bacteria of the family Pseudomonadaceae, in particular of the genera Pseudomonas and Xanthomonas, bacteria of the family Enterobacteriaceae, in particular of the genera Escherichia, Klebsiella, Proteus, Providencia, Salmonella, Serratia and Shigella, bacteria of the family Pasteurellaceae, in particular of the genus Haemophilus, bacteria of the family Micrococcaceae, in particular of the genera Micrococcus and Staphylococcus, bacteria of the family Streptococcaceae, in particular of the genera Streptococcus and Enterococcus and bacteria of the family Bacillaceae, in particular of the genera Bacillus and Clostridium, and in the eradication of Helicobacter in ulcers of the gastrointestinal tract.

12. Use according to claim 10 for preparing a pharmaceutical preparation for the treatment of infections which are caused by viruses which are selected from the group which consists of viruses of the genus Parvoviridae, in particular parvo viruses, dependoviruses, densoviruses, viruses of the genus Adenoviridae, in particular adenoviruses, mastadenoviruses, aviadenoviruses, viruses of the genus Papovaviridae, in particular papovaviruses, in particular papillomaviruses ("wart" viruses), polyomaviruses, in particular JC virus, BK virus and miopapovaviruses, viruses of the genus Herpesviridae in particular herpes simplex viruses, varicella-zoster viruses, human cytomegalovirus, Epstein-Barr viruses, human herpesvirus 6, human herpesvirus 7, human herpesvirus 8, viruses of the genus Poxviridae, in particular poxviruses, orthopoxviruses, parapoxviruses, molluscum contagiosum virus, aviviruses, capriviruses, leporipoxviruses, primarily hepatotropic viruses, in particular hepatitisviruses, such as hepatitis A viruses, hepatitis B viruses, hepatitis C viruses, hepatitis D viruses, hepatitis E viruses, hepatitis F viruses, hepatitis G viruses, hepadnaviruses, in particular all hepatitisviruses, such as hepatitis B virus, hepatitis D viruses, viruses of the genus Picornaviridae, in particular picornaviruses, all enteroviruses, all polioviruses, all coxsackie-viruses, all echoviruses, all rhinoviruses, hepatitis A virus, aphtho viruses, viruses of the genus Calciviridae, in particular hepatitis E viruses, viruses of the genus Reoviridae, in particular reoviruses, orbiviruses, rotaviruses, viruses of the genus Togaviridae, in particular togaviruses, alphaviruses, rubiviruses, pestiviruses, rubellavirus, viruses of the genus Flaviviridae, in particular flaviviruses, FSME virus, hepatitis C virus, viruses of the genus Orthomyxoviridae, in particular all influenza viruses, viruses of the genus Paramyxoviridae, in particular paramyxoviruses, morbillivirus, pneumovirus, measles virus, mumps virus, viruses of the genus Rhabdoviridae, in particular rhabdoviruses, rabies virus, lyssavirus, vascular stomatitisvirus, viruses of the genus Coronaviridae, in particular coronaviruses, viruses of the genus Bunyaviridae, in particular bunyaviruses, nairo virus, phlebovirus, uukuvirus, hantavirus, hantaan virus, viruses of the genus Arenaviridae, in particular arenaviruses, lymphocytic choriomeningitis virus, viruses of the genus Retro viridae, in particular retroviruses, all HTL viruses, human T-cell leukaemia virus, oncomaviruses, spumaviruses, lentiviruses, all HI viruses, viruses of the genus Filoviridae, in particular Marburg and Ebola virus, slow-viruses, prions, oncoviruses and leukaemia viruses.

13. Use according to claim 10 for preparing a pharmaceutical preparation for prevention and treatment of infections caused by unicellular parasites, namely the causative organisms of malaria, of sleeping sickness, of Chagas' disease, of toxoplasmosis, of amoebic dysentery, of leishmaniases, of trichomoniasis, of pneumocystosis, of balantidiasis, of cryptosporidiosis, of sarcocytosis, of acanthamoebosis, of naeglerosis, of coccidiosis, of giardiasis and of lambliasis.

## Revendications

1. Composés organophosphorés de formule générale (I) 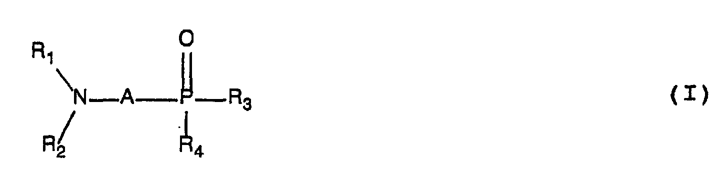 dans laquelle R₁ et R₂ sont identiques ou différents et sont choisis dans le groupe constitué de l'hydrogène, d'un alkyle en C₁₋₉ substitué ou non substitué, d'un hydroxy- alkyle en C₁₋₉ substitué ou non substitué, d'un alcényle en C₁₋₉ substitué ou non substitué, d'un alkinyle en C₁₋₉ substitué ou non substitué, d'un aryle substitué ou non substitué, d'un acyle substitué ou non substitué, d'un cycloalkyle substitué ou non substitué, d'un aralkyle substitué ou non substitué, d'un reste hétérocyclique substitué ou non substitué, d'un halogène, d'OX₁ et OX₂,
où X₁ et X₂ peuvent être identiques ou différents et sont choisis dans le groupe constitué de l'hydrogène, d'un alkyle en C₁₋₉ substitué ou non substitué, d'un hydroxyalkyle en C₁₋₉ substitué ou non substitué, d'un alcényle en C₁₋₉ substitué ou non substitué, d'un alkinyle en C₁₋₉ substitué ou non substitué, d'un aryle substitué ou non substitué, d'un acyle substitué ou non substitué, d'un cycloalkyle substitué ou non substitué, d'un aralkyle substitué ou non substitué, d'un reste hétérocyclique substitué ou non substitué,
dans laquelle A répond à la formule suivante (II) : 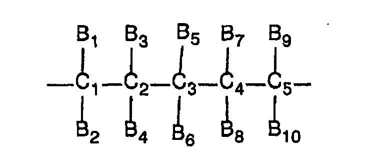 où un ou plusieurs des atomes de carbone, choisis dans le groupe C₃, C₄, C₅, avec leurs substituants qui peuvent aussi être supprimés, et au moins un substituant présent de B₁ à B₁₀ est un groupe cycloalkyle en C₃₋₈ alkyle en C₀₋₉, où aussi bien le groupe cycloalkyle en C₃₋₈ que le groupe alkyle en C₀₋₉ peuvent présenter une ou plusieurs doubles liaisons et un ou deux atomes de carbone du groupe cycloalkyle peuvent être remplacés par des atomes d'azote, d'oxygène ou de soufre, et où aussi bien le groupe cycloalkyle que le groupe alkyle peuvent être substitués par des groupes hydroxy, halogène, amino, oxo, par des groupes alkyle en C₁₋₉ et alcényle en C₂₋₉ ramifiés ou non ramifiés, où les groupes alkyle en C₁₋₉ et alcényle en C₂₋₉ peuvent être substitués par des groupes hydrogène, hydroxy, amino, halogène et oxo, et les substituants B₁ à B₁₀ présents qui restent sont choisis dans le groupe constitué par l'hydrogène, l'hydroxy, un halogène, un amino, les restes alkyle en C₁₋₂₆, les restes alcoxy en C₁₋₂₆, les restes alcoxy en C₁₋₂₆ alkyle en C₁₋₂₆ ou les deux substituants d'un atome de C forment ensemble un groupe oxo, où chaque reste alkyle en C₁₋₂₆ et chaque reste alcoxy en C₁₋₂₆ peut être ramifié ou non ramifié et saturé ou insaturé avec une ou plusieurs doubles liaisons et peuvent être substitués par des groupes hydroxy, amino, halogène et oxo,
dans laquelle R₃ et R₄ sont identiques ou différents et sont choisis dans le groupe constitué d'un alkyle en C₁₋₂₆ substitué ou non substitué, d'un hydroxyalkyle en C₁₋₂₆, d'un aryle substitué ou non substitué, d'un acyle substitué ou non substitué, d'un aralkyle substitué ou non substitué, d'un alcényle en C₁₋₂₆ substitué ou non substitué, d'un alkinyle en C₁₋₂₆ substitué ou non substitué, d'un cycloalkyle substitué ou non substitué, d'un reste hétérocyclique substitué ou non substitué, d'un halogène, d'OX₃ et OX₄,
où X₃ et X₄ sont identiques ou différents et sont choisis dans le groupe constitué de l'hydrogène, d'un alkyle en C₁₋₂₆ substitué ou non substitué, d'un hydroxyalkyle en C₁₋₂₆ substitué ou non substitué, d'un aryle substitué ou non substitué, d'un aralkyle substitué ou non substitué, d'un alcényle en C₁₋₂₆ substitué ou non substitué, d'un alkinyle en C₁₋₂₆ substitué ou non substitué, d'un cycloalkyle substitué ou non substitué, d'un reste hétérocyclique substitué ou non substitué, d'un silyle, d'un cation de base organique ou inorganique, en particulier d'un métal du premier, deuxième ou troisième groupe principal du système périodique, de l'ammonium, de l'ammonium et des composés de l'ammonium substitués, qui sont dérivés de l'éthylènediamine ou d'aminoacides, et leurs sels, esters et sels d'ester pharmaceutiquement acceptables.

2. Composé selon la revendication 1, **caractérisé en ce que** les composés organophosphorés répondent à la formule (II) 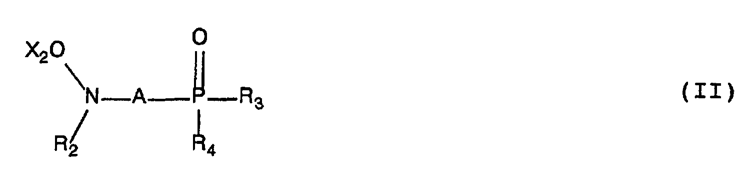 où
X₁ est l'hydrogène et R₂ est un reste acyle, particulièrement de préférence un reste formyle ou un reste acétyle, et R₃, R₄ et A ont la même signification que dans la formule (I).

3. Composé selon la revendication 1 ou la revendication 2, **caractérisé en ce que** X₃ et X₄ sont choisis dans le groupe constitué de OX₃ et OX₄, et X₃ et X₄ sont choisis dans le groupe constitué de l'hydrogène, d'un métal du premier, deuxième ou troisième groupe principal du système périodique, de l'ammonium, de l'ammonium et des composés de l'ammonium substitués, qui sont dérivés de l'éthylènediamine ou d'aminoacides.

4. Composé selon l'une des revendications précédentes, **caractérisé en ce que** la chaîne carbonée de A avec la formule (II) est constituée des trois atomes de carbone C₁, C₂, C₃.

5. Composé selon l'une des revendications 1 à 3, **caractérisé en ce que** B₁ et B₂ ensemble ou B₇ et B₈ forment ensemble un groupe oxo et **en ce que** la chaîne carbonée dans A est constituée des quatre atomes de carbone C₁, C₂, C₃, C₄.

6. Composé selon l'une des revendications 1 à 3, **caractérisé en ce que** la chaîne carbonée de A avec la formule (II) est constituée des quatre atomes de carbone C₁, C₂, C₃, C₄ et B₇ ou B₈, ou les deux, sont un groupe hydroxy.

7. Composé selon l'une des revendications 1 à 3, **caractérisé en ce que** la chaîne carbonée est constituée de préférence des cinq atomes de carbone C₁, C₂, C₃, C₄, C₅, où B₁ et B₂ forment ensemble un groupe oxo et B₉ ou B₁₀ sont un groupe hydroxy ou B₉ et B₁₀ forment également ensemble un groupe oxo.

8. Composé selon la revendication 6 ou la revendication 7 qui dépend de la revendication 1 ou 2, **caractérisé en ce que** R₃ ou R₄ ou les deux sont des groupes méthylène.

9. Utilisation de composés organophosphorés selon l'une des revendications 1 à 8 pour la fabrication d'un médicament pour le traitement de processus infectieux chez l'homme et l'animal, qui sont provoqués par des virus, des bactéries, des champignons ou des parasites et comme fongicide, bactéricide ou herbicide chez les plantes.

10. Utilisation selon la revendication 9 pour la fabrication d'un médicament pour le traitement des infections, causées par des bactéries, des virus, des champignons ou des parasites uni- ou pluricellulaires.

11. Utilisation selon la revendication 10 pour la fabrication d'un médicament pour le traitement des infections, qui sont provoquées par des bactéries, qui sont choisies dans le groupe constitué des bactéries de la famille des Propionibacteriaceae, en particulier du genre des Propionibacterium, en particulier de l'espèce Propionibacterium acnés, des bactéries de la famille des Actinomycetaceae, en particulier du genre des Actinomyces, des bactéries du genre des Corynebacterium, en particulier des espèces Corynebacterium diphteriae et Corynebacterium pseudotuberculosis, des bactéries de la famille des Mycobacteriaceae, du genre des Mycobacterium, en particulier des espèces Mycobacterium leprae, Mycobacterium tuberculosis, Mycobacterium bovis et Mycobacterium avium, des bactéries de la famille des Chlamydiaceae, en particulier des espèces Chlamydia trachomatis et Chlamydia psittaci, des bactéries du genre des Listeria, en particulier de l'espèce Listeria monocytogenes, des bactéries de l'espèce Erysipelthrix rhusiopathiae, des bactéries du genre des Clostridium, des bactéries du genre des Yersinia, des espèces Yersinia pestis, Yersinia pseudotuberculosis, Yersinia enterocolitica et Yersinia ruckeri, des bactéries de la famille des Mycoplasmataceae, des genres des Mycoplasma et des Ureaplasma, en particulier des espèces Mycoplasma pneumoniae, des bactéries du genre des Brucella, des bactéries du genre des Bordetella, des bactéries de la famille des Neisseriaceae, en particulier des genres des Neisseria et des Moraxella, en particulier des espèces Neisseria meningitides, Neisseria gonorrhoeae et Moraxella bovis, des bactéries de la famille des Vibrionaceae, en particulier des genres des Vibrio, des Aeromonas, des Plesiomonas et des Photobacterium, en particulier des espèces Vibrio cholerae, Vibrio anguillarum et Aeromonas salmonicidas, des bactéries du genre des Campylobacter, en particulier des espèces Campylobacter jejuni, Campylobacter coli et Campylobacter fetus, des bactéries du genre des Helicobacter, en particulier de l'espèce Helicobacter pylori, des bactéries des familles des Spirochaetaceae et des Leptospiraceae, en particulier des genres des Treponema, des Borrelia et des Leptospira, en particulier Borrelia burgdorferi, des bactéries du genre des Actinobacillus, des bactéries de la famille des Legionellaceae, du genre des Legionella, des bactéries de la famille des Rickettsiaceae et de la famille des Bartonellaceae, des bactéries des genres des Nocardia et des Rhodococcus, des bactéries du genre des Dermatophilus, des bactéries de la famille des Pseudomonadaceae, en particulier des genres des Pseudomonas et des Xanthomonas, des bactéries de la famille des Enterobacteriaceae, en particulier des genres des Escheridia, des Klebsiella, des Proteus, des Providencia, des Salmonella, des Serratia et des Shigella, des bactéries de la famille des Pasteurellaceae, en particulier du genre des Haemophilius, des bactéries de la famille des Micrococcaceae, en particulier des genres des Micrococcus et des Staphylococcus, des bactéries de la famille des Streptococcaceae, en particulier des genres des Streptococcus et des Enterococcus et des bactéries de la famille des Bacillaceae, en particulier des genres des Bacillus et des Clostridium, et pour la thérapie d'éradication d'Helicobacter en cas d'ulcères gastrointestinaux.

12. Utilisation selon la revendication 10 pour la fabrication d'un médicament pour le traitement des infections, qui sont provoquées par des virus, qui sont choisis dans le groupe constitué des virus du genre des Parvoviridae, en particulier les Parvovirus, les Dependovirus, les Densovirus, des virus du genre des Adenoviridae, en particulier les Adenovirus, les Mastadenovirus, les Aviadenovirus, des virus du genre des Papovaviridae, en particulier les Papovavirus, en particulier les Papillomavirus (appelés virus papilloma), les Polyomavirus, en particulier le virus JC, le virus BK, et les Miopapovirus, des virus du genre des Herpesviridae, en particulier les Herpes-Simplex-virus, les virus de la varicelle/Zoster, du Zytomegalievirus humain, du virus d'Epstein-Barr, du virus de l'Herpes 6 humain, du virus de l'Herpes 7 humain, du virus de l'Herpes 8 humain, des virus du genre des Poxviridae, en particulier les Pockenvirus, de l'Orthopox-Parapox-, Molluscum-Contagiosum-Virus, des Avivirus, des Caprivirus, des Leporipoxvirus, des virus hépatotropiques primaires, en particulier les virus d'hépatite, comme les virus de l'hépatite A, les virus de l'hépatite B, les virus de l'hépatite C, les virus de l'hépatite D, les virus de l'hépatite E, les virus de l'hépatite F, les virus de l'hépatite G, les Hepadnavirus, en particulier tous les virus d'hépatite, comme le virus de l'hépatite B, les virus de l'hépatite D, des virus du genre des Picornaviridae, en particulier les Picornavirus, tous les Enterovirus, tous les Poliovirus, tous les Coxsackievirus, tous les Echovirus, tous les Rhinovirus, le virus de l'hépatite A, les Aphtovirus, des virus du genre des Calciviridae, en particulier les virus de l'hépatite B, des virus du genre des Reoviridae, en particulier les Reovirus, les Orbivirus, les Rotavirus, des virus du genre des Togaviridae, en particulier les Togavirus, les Alphavirus, les Rubivirus, les Pestivirus, le Rubellavirus, des virus du genre des Flaviviridae, en particulier les Flavivirus, le virus FMSE, le virus de l'hépatite C, des virus du genre des Orthomyxoviridae, en particulier les Influenzavirus, des virus du genre des Paramyxoviridae, en particulier les Paramyxovirus, le Morbillivirus, le Pneumovirus, le virus de la rougeole, le virus des oreillons, des virus du genre des Rhabdoviridae, en particulier les Rhabdovirus, le Rabiesvirus, le Lyssavirus, le Stomatitisvirus vasculaire, des virus du genre des Coronaviridae, en particulier les Coronavirus, des virus du genre des Bunyaviridae, en particulier les Bunyavirus, le Nairovirus, le Phlebovirus, l'Uukuvirus, l'Hantavirus, l'Hantaanvirus, de virus du genre des Arenaviridae, en particulier les Arenavirus, le Choriomeningitis-virus lymphocytaire, des virus du genre des Retroviridae, en particulier les Retrovirus, tous les virus HTL, le virus de la leucémie des cellules-T humaines, les Oncornovirus, les Spumavirus, les Lentivirus, tous les virus HI, des virus du genre des Filoviridae, en particulier le virus Marburg et le virus Ebola, les « Slowvirus », les Prions, les Oncovirus et les virus de la leucémie.

13. Utilisation selon la revendication 10 pour la fabrication d'un médicament pour la prévention et le traitement des infections causées par des parasites monocellulaires, à savoir l'agent de la malaria, de la maladie du sommeil, de la maladie de Chagas, de la toxoplasmose, de la dysenterie amibienne, de la leishmaniose, de la trichomoniasis, de la pneumosystose, de la balantidiose, de la kryptosporidiose, de la sarkosystose, de la « akanthamöbose », de la naegierose, de la coccidiose, de la giardiose et de la lambliose.
